(19) Europäisches Patentamt
European Patent Office
Office européen des brevets

(11) EP 0 471 687 B1

(12) EUROPEAN PATENT SPECIFICATION

(45) Date of publication and mention of the grant of the patent:
**10.12.1997 Bulletin 1997/50**

(21) Application number: **90906539.3**

(22) Date of filing: **04.04.1990**

(51) Int Cl.[6]: **C12N 15/54**, C12N 1/21, C12N 9/10, C12N 15/52, C12N 15/31, C12N 15/74

(86) International application number:
**PCT/US90/01811**

(87) International publication number:
**WO 90/12098 (18.10.1990 Gazette 1990/24)**

# (54) METHODS AND NUCLEIC ACID SEQUENCES FOR THE EXPRESSION OF THE CELLULOSE SYNTHASE OPERON

VERFAHREN UND NUKLEINSÄURESEQUENZEN ZUR EXPRESSION DES ZELLULOSE-SYNTHASE-OPERONS

PROCEDES ET SEQUENCES D'ACIDES NUCLEIQUES POUR L'EXPRESSION DE L'OPERON DE SYNTHASE DE LA CELLULOSE

(84) Designated Contracting States:
**AT BE CH DE DK ES FR GB IT LI LU NL SE**

(30) Priority: **12.04.1989 US 337194**
**23.03.1990 US 496236**

(43) Date of publication of application:
**26.02.1992 Bulletin 1992/09**

(73) Proprietor: **MONSANTO COMPANY**
**St. Louis, Missouri 63167 (US)**

(72) Inventors:
- **BEN-BASSAT, Arie**
  **Walnut Creek, CA 94598 (US)**
- **CALHOON, Roger, D.**
  **Concord, CA 94518 (US)**
- **FEAR, Anna, Lisa**
  **Oakland, CA 94611 (US)**
- **GELFAND, David, H.**
  **Oakland, CA 94611 (US)**
- **MEADE, James, H.**
  **El Sobrante, CA 94803 (US)**
- **TAL, Rony**
  **Richmond, CA 94805 (US)**
- **WONG, Hing**
  **San Ramon, CA 94585 (US)**
- **BENSIMAN, Moshe**
  **91 999 Jerusalem (IL)**

(74) Representative: **Bizley, Richard Edward et al**
**Hepworth, Lawrence, Bryer & Bizley**
**Merlin House**
**Falconry Court**
**Baker's Lane**
**Epping Essex CM16 5DQ (GB)**

(56) References cited:
- **Agricultural and Biological Chemistry, vol. 49, no. 7, July 1985; Tokyo, JP; M. Fukaya et al, pages 2083-2090**
- **Applied and Environmental Microbiology, vol.55, no. 1, January 1989; American Society for Microbiology, M. Fukaya et al, pages 171-176.**
- **The Journal of Biological Chemistry, vol. 258, no. 7, 10 April 1983; US; Y. Aloni et al, pp. 4419-4423**
- **Proc. Natl. Acad. Sci USA, vol. 84, October 1987; T. E. Bureau et al, pages 6985-6989.**
- **Chemical Abstracts, vol. 105, 1986; Columbus, Ohio US; M.P. Thelan et al, page 295**
- **The Journal of Biol. Chem., vol. 265, no. 9, 25 March 1990; F.C. Lin et al, pages 4782-4784**
- **Agricultural and Biological Chemistry, vol. 49, no. 5, 1985; M. Fukaya et al, pages 1349-1355**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

TECHNICAL FIELD OF THE INVENTION

The invention relates to the field of recombinant DNA techniques for the production of proteins. More specifically, this invention relates to the cloning of the bacterial cellulose synthase operon, the expression of this operon and methods of using this operon for the production of cellulose in recombinant microorganisms.

BACKGROUND OF THE INVENTION

Acetobacter is characteristically a Gram-negative, rod-shaped bacterium 0.6-0.8 um by 1.0-4 um. It is strictly aerobic; metabolism is respiratory, never fermentative. It is further distinguished by the ability to produce multiple poly B (1-4)-glucan chains, chemically identical to cellulose. Multiple cellulose chains or microfibrils are synthesized at the bacterial surface at sites on the cell wall. The production of cellulose by Acetobacter has been the subject of intense study since at least the 1930's. In particular, Acetobacter xylinum has been widely studied to attempt to elucidate the mechanism of cellulose synthesis in intact cells [Schramm and Hestrin, (1954) J. Gen. Microbiol. 11:123-129].

The enzymatic pathway for cellulose synthesis in Acetobacter xylinum has been investigated and essentially four enzymatic steps have been characterized in cell-free extracts of A. xylinum which appear to comprise the complete pathway from glucose to cellulose. These are the phosphorylation of glucose by glucokinase [Benziman, et al., (1972) J. Bacterial., 111:325-330], the isomerization of glucose-6-phosphate to glucose-1-phosphate by phosphoglucomutase {Gromet, e al., (1957) Biochem. J., 67:679-689; Frei-Roitman, Factors affecting the activity of phosphoglucomutase and UDP-glucose pyrophosphorylase of Acetobacter xylinum, M.Sc. thesis, the Hebrew University of Jerusalem, Jerusalem, Israel (1974)]; the synthesis of uridine 5'-diphosphoglucose (UDP-glc) by UDPG-pyrophosphorylase, [Frei-Roitman, supra; Swisa, Biosynthesis of cellulose in Acetobacter xylinum, Ph.D. thesis, the Hebrew University of Jerusalem, Jerusalem, Israel (1978)], and the cellulose synthase reaction.

Attempts to purify cellulose synthase from a strain of A. xylinum employing conventional chromatographic techniques have not been especially successful, but recently the enzyme has been significantly purified (P. Ross and M. Benziman (1989) in Biosynthesis and Biodegradation of Cellulose and Cellulose Materials, eds. Weimar and Higler, Marcel Dekker, Inc. NY, in press), and its properties and structure in the purified state are currently under investigation.

Similarly, attempts to purify cellulose synthase by in vitro cellulose entrapment and chromatographic techniques have resulted in a partially purified 83 kilodalton (kd) polypeptide (Lin and Brown, The Tenth Cellulose Conference, May 29-June 2, 1988, Abstract BG1, page 27).

A more complete knowledge of the biochemistry of cellulose synthesis would facilitate greater productivity and yield of cellulose from cultures of cellulose-producing microorganisms. The growth of bacterial cells in culture is observed to be initially exponential but slows as the cells enter a stationary growth phase. The majority of cellulose is produced later in fermentation when the number of cells is highest, however the amount of cellulose made per cell per unit time (specific productivity) declines as the fermentation proceeds. It is believed that cellulose synthase activity may be rate limiting as cells in culture reach the stationary growth phase. One improvement in cellulose production would be to remove a rate-limiting step in cellulose synthesis, thereby preventing the observed decline in cellulose specific productivity in culture.

The ability to produce recombinant cellulose synthase provides an important tool useful in exploring the mechanisms of cellulose synthesis, ultimately providing enhanced cellulose production from bacterial culture.

In first aspect, the present invention provides an isolated native, cloned recombinant or synthetic polynucleotide sequence encoding a bacterial cellulose synthase operon, wherein with reference to Figure 1 said polynucleotide comprises four contiguous genes, the first gene corresponding substantially to nucleotides 328 to 2589, the second gene corresponding substantially to nucleotides 2594 to 4999, the third gene corresponding to substantially nucleotides 5005 to 8961, and the fourth gene corresponding substantially to nucleotides 8964 to 9431.

The polynucleotide sequence may be one derived from the genome of Acetobacter. Also, the polynucleotide sequence may further comprise promoter located adjacent to and upstream from said operon.

In second aspect the invention provides an isolated native, cloned recombinant or synthetic polynucleotide substantially corresponding to the nucleotide sequence for cellulose synthase A, cellulose synthase B, cellulose synthase C, or cellulose synthase D, in each case as shown in Figure 1. This polynucleotide is preferably operatively linked to a control sequence for expression and may further comprisea promoter located adjacent to and upstream from said polynucleotide corresponding to the nucleotide sequence for cellulose synthase A, B, C or D.

In a third aspect the invention provides recombinant host cells transformed with a polynucleotide of the invention.

A fourth aspect of the invention provides a method for producing bacterial cellulose synthase comprising culturing cells transformed with a polynucleotide of the invention under conditions suitable for the expression of bacterial cellulose synthase, and recovering the expressed bacterial cellulose synthase from the culture. Accordingly, isolated recombinant protein encoded by a polynucleotide of the invention is provided as a fifth aspect.

Expression vector constructions of the invention can either replicate independently or may be designed so as to introduce a heterologous promoter into the Acetobacter chromosome, thereby replacing the native cellulose synthase operon promoter.

A sixth aspect of the invention providesa method for increasing cellulose production in a recombinant microorganism, which method comprises:

(a) transforming a suitable microorganism with a vector comprising at least one gene substantially corresponding to the nucleotide sequence for cellulose synthase A, cellulose synthase B, cellulose synthase C, or cellulose synthase D, in each case as shown in Figure 1, and
(b) culturing said transformed microorganism under conditions suitable for production of cellulose.

As discussed above, the chromosomal cellulose synthase promoter may be replaced with the heterologous promoter to overexpress the cellulose synthase operon at the chromosomal level.

The isolated recombinant cellulose synthase B protein is capable of synthesizing β(1-4)-glucan polymers from uridine 5'-diphosphoglucose, whereas the protein encoded by gene A is capable of complementing cellulose negative Acetobacter cells defective in both cellulose synthase and diguanylate cyclase activities. The protein encoded by either gene C or D is capable of synthesizing β(1-4)-glucan polymers from uridine 5'-diphosphoglucose and secreting the product from the cells in vivo when the respective protein is combined in appropriate proportions with the other three proteins of the cellulose synthase operon.

A seventh aspect of the invention provides a recombinant DNA vector comprising:

(a) at least one gene derived from the cellulose synthase operon, said gene comprising a sequence substantially corresponding to the nucleotide sequence for cellulose synthase A, cellulose synthase B, cellulose synthase C, or cellulose synthase D, in each case as shown in Figure 1,

(b) A functional Acetobacter origin of replication-containing fragment of that 3.5 kb plasmid DNA which is obtainable by SacI digestion of ATCC 67925 or ATCC 67926, and

(c) one or more DNA segments that convey resistance to at least one antibiotic when transformed into a sensitive host cell that is susceptible to transformation, cell division and culture.

The recombinant DNA vector is preferably that vector obtainable from ATCC 67925 or ATCC 67926, or the vector may be identical to that preferred vector except that the cloned 8.3 kb SmaI restriction fragment is in the reverse orientation. The vectors may be developed into shuttle vectors for use in cloning DNA in bacterial host cells, such as E. coli.

Figure 1 is the nucleic acid sequence and deduced amino acid sequence of the cellulose synthase operon. The cellulose synthase operon is approximately 9217 bp in length and consists of four genes, a 2261 nucleotide sequence designed A (nucleotides 328-2589), a 2405 nucleotide sequence designated B (nucleotides 2594-4999), a 3956 nucleotide sequence designated C (nucleotides 5005 to 8961) and a 467 nucleotide sequence designated D (nucleotides 8964 to 9431). The nucleotide sequences of each gene provided herein may include signal sequences. The mature protein encoded by its respective gene may have undergone processing and if so, the corresponding gene sequence will be shorter than that provided above. For example, the alanine codon which corresponds to the first amino acid of the purified cellulose synthase B protein is flanked by two upward arrows, ↑↑. The site of transcription initiation is designed by a downward arrow, ↓, positioned over the A at nucleotide 235. The underlined nucleotide sequence following gene D designates the transcription terminator region comprising an inverted repeat sequence characteristic of stem-and-loop structures. The sequence of oligonucleotide MK170 is indicated above nucleotides 2190 to 2210, and that of MK172 is indicated above nucleotides 4564 to 4583.

Figure 2 depicts the construction of cosmid vector pKT230cos5.

Figure 3 is an illustration of the construction of TRT18-1 which contains the full-length cellulose synthase B gene.

Figure 4 is a restriction map of plasmid pUC18-824.

Figure 5 is a restriction map of the 8.3 kb SmaI fragment and the 7.2 kb BamHI fragment from cosmid T5A1.

Figure 6 is a restriction and functional map of plasmid pUC18-824 FS6; pUC18-824 FS1 is homologous to pUC18-824 FS6 except the orientation of the SmaI restriction fragment carrying the cellulose synthase gene is reversed.

Figure 7 is a restriction and functional map of plasmid pUC19-824.

Figure 8 schematically illustrates the construction of plasmid pABCD.

Figure 9 is a flow chart describing the construction of expression vectors containing heterologous promoters to transcribe the genes of the cellulose synthase operon.

## Detailed Description of the Invention

In order that the invention herein described may be more fully understood, the following detailed description is set forth.

### A. Definitions

As used herein, the term "cellulose synthase" refers to one or more polypeptides associated in the in vivo biochemical conversion of uridine 5'-diphosphoglucose to bacterial cellulose and secretion outside of the cell. A single transcriptional unit containing four genes associated with the synthesis of cellulose synthase is encoded by the nucleic acid sequence provided in Figure 1.

The term "cellulose synthase gene" is defined as a nucleic acid sequence encoding a polypeptide product associated with the cellulose synthase operon. The term is not limited to any Acetobacter bacterial strain or species.

"Cellulose synthase operon," as used herein, refers to a stretch of DNA sequence which codes for a group of protein products associated with cellulose synthesis and secretion outside of the cell. Optionally, the operon may include transcriptional elements such as a promoter region and a transcription terminator region which regulate the expression of the genes encoding the proteins.

"Cellulose synthase activity" is defined by the ability to synthesize cellulose [β(1-4)-glucan] from UDP-glc. This activity is measured in vitro by incorporation of UDA-($^{14}C$) glucose to cellulose (base insoluble) and is measured as nmole (nanomole) of glucose incorporated to cellulose per min.

"Cellulose synthase specific activity" is defined as nmole glucose incorporated to cellulose/ min/mg protein. Cellulose synthase specific activity in Acetobacter cells is normally ranged from 0.2 to 4.0 nmole glc/min/mg cell protein.

As used herein, the term "Acetobacter" refers to a genus of microorganisms, and in particular, to members of that genus which produce cellulose.

"Suitable microorganism" refers to a microorganism which is capable of producing cellulose when transformed with one or more of the genes associated with the cellulose synthase operon. Suitable micororganisms include those host cells which are capable of cellulose production in the absence of transformation, or those host cells which are deficient in one or more of the genes whose activity may be replaced by at least one gene of the cellulose synthase operon.

"Operably linked" refers to a juxtaposition such that the normal function of the components can be performed. Thus, a coding sequence "operably linked" to control sequences refers to a configuration wherein the coding sequences can be expressed under the control of these sequences. Such control may be direct, that is, a single gene associated with a single promoter, or indirect, as in the case where a polycistronic transcript is expressed from a single promoter.

"Control sequence" refers to a DNA sequence or sequences necessary for the expression of an operably linked coding sequence in a particular host organism. The control sequences which are suitable for procaryotes, for example, include a promoter, optionally an operator sequence, a ribosome binding site, a transcription terminator, and possibly other as yet poorly understood sequences. Eucaryotic cells are known to utilize promoters, polyadenylation signals, and enhancers.

"Cells" or "recombinant host cells" or "host cells" are often used interchangeably, and all such designation include progeny. Thus, "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny which have the same functionality as screened for in the originally transformed cell are included. Where distinct designations are intended, it will be clear from the context.

As used herein, a polynucleotide "derived from" a designated sequence, for example, the DNA from the cellulose synthase B gene, refers to a polynucleotide sequence which is comprised of a sequence of at least 6-20 nucleotides, more preferably at least 15 to 20 nucleotides corresponding, i.e., identical to or complementary to, a region of the designated nucleotide sequence. The correspondence to the nucleic acid sequence will be approximately 70% or greater, will preferably be at least 80%, and even more preferably will be at least 90%.

The correspondence or non-correspondence of the derived sequence to other sequences can be determined by hybridization under the appropriate stringency conditions, using standard DNA hybridization technologies in liquid phases or on solid supports. Hybridization techniques for determining the complementarity of nucleic acid sequences are known in the art (see, for example, Sambrook et al. (1989) Molecular Cloning: A Laboratory Manual, Second Edition, Cold Spring Harbor Press, Cold Spring Harbor, NY). In addition, mismatches of duplex polynucleotides formed by hybridization can be determined by known techniques, including digestion with a nuclease such as S1, that specifically digests single-stranded sequences in duplex polynucleotides.

The derived polynucleotide is not necessarily physically derived from the nucleotide sequence shown, but may be generated in any manner, including for example, chemical synthesis, DNA replication or reverse transcription, which methods are based on the information provided by the sequence of bases in the region(s) from which the polynucleotide is derived.

Similarly, a polypeptide "derived from" a designated sequence, for example, from cellulose synthase B, refers to a polypeptide having an amino acid sequence identical to that of a polypeptide encoded in the sequence or a protein thereof wherein the portion consists of at least 5-10 amino acids, and more preferably at least 10-15 amino acids, which is immunologically identifiable with a polypeptide encoded in the sequence, or exhibits similar biological activity as that of the reference protein in the in vitro or in vivo assays described herein.

As used herein with reference to an amino acid sequence "substantial correspondence" refers to a sequence usually differing by fewer than 10 amino acids, more usually differing by fewer than 5 amino acids. The recombinant protein, whether "A", "B", "C" or "D", display substantially the same biological properties as the naturally occurring protein. The biological properties include immunological properties, where antibodies raised to the authentic protein cross-react with the recombinant protein.

The term "recombinant polypeptide" as used herein to characterize a polypeptide useful for the production of cellulose synthase intends a polypeptide encoded by genomic, cDNA, semisynthetic, or synthetic nucleic acid sequences which, by virtue of their origin or manipulation: (1) are not associated with all or a portion of the polynucleotide with which they are associated in nature or in the form of a library; and/or (2) are linked to a polynucleotide other than that to which it is linked in nature.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may also be integrated into the host chromosome.

"Sensitive host cell" refers to a host cell that cannot grow in the presence of a given antibiotic without a DNA segment containing a gene that confers resistance thereto.

As used herein, the term "vector" refers to a polynucleotide suitable for transferring nucleic acid sequences into a host cell. The term may include plasmid, mini-chromosomes, phage, naked DNA and the like.

B. General Description

The methods illustrated below to obtain one or more DNA sequences encoding genes of the bacterial cellulose synthase operon are merely for purposes of illustration and are typical of those that might be used. However, once the genes have been identified, other procedures may also be employed, as is understood in the art.

Obtaining the Coding Sequences for the Bacterial Cellulose Synthase Operon

The polynucleotide encoding the bacterial cellulose synthase operon was obtained from an Acetobacter DNA library, as set forth in the examples, and genetic complementation was used to identify the genes.

The procedures for obtaining the nucleotide sequence encoding the bacterial cellulose synthase operon employ number of individual steps that required adaptation for Acetobacter DNA, and include (1) preparation and characterization of cellulose negative (Cel) mutant Acetobacter strains; (2) construction of appropriate vectors for cloning; (3) construction of an Acetobacter DNA library; (4) identification and isolation of DNA insert sequences capable of restoring cellulose synthase activity in the Cel Acetobacter mutants; (5) mapping and subcloning of the nucleotide sequences encoding the bacterial cellulose synthase operon for both sequence analysis and localization of the cellulose synthase coding sequences; and (6) cloning and expression of the DNA encoding the products of the cellulose synthase operon.

Complementation studies indicate that strains that are defective in cellulose synthase activity can be complemented by gene B, and that cellulose negative mutants that were defective in both cellulose synthase and diguanylate cyclase activities are complemented by gene A. Cellulose negative class III mutants are complemented by a DNA fragment that codes for genes C and D. The mutants in this group make cellulose in vitro and have all the enzymatic activities necessary for cellulose production. Gene D encodes a protein that is associated with cellulose synthesis. Disruption of this gene significantly reduces cellulose synthesis.

Genes A, C and D may code for regulatory, structural, membrane bound or processing proteins required in cellulose synthesis in vivo. The availability of coding sequences for their respective gene product permits the synthesis of large amounts of each protein for studies to further elucidate the mechanism of cellulose synthesis.

Expression of Bacterial Cellulose Synthase

According to one method of the invention, the polynucleotide encoding the cellulose synthase operon may be cloned into an appropriate vector, transformed into a suitable microorganism host and cultured under conditions which permit the expression of cellulose synthase. Alternatively, given the sequence identity of each gene in this operon, each gene may be independently cloned and expressed to produce the desired gene product.

Transcription of the polynucleotide sequences encoding the cellulose synthase operon gene products may be performed using the endogenous Acetobacter promoter or, alternatively, may be driven by heterologous bacterial promoters, including those derived from E. coli or B. subtilis. Many of the heterologous promoters described herein, such as the lac, trp and tac are regulated promoters and are therefore useful in the method of the invention to control the expression of the bacterial cellulose synthase operon. However, promoters such as the $P_L$ promoters, which are regulated by temperature sensitive repressors, i.e., nonfunctional beyond 37°C, would not be operable in many Acetobacter strains since these strains are normally cultured at 35°C or lower and would not grow beyond this temperature range.

Yet another means to control expression of the cellulose synthase operon may employ a heterologous transcription terminator to stabilize the mRNA transcript. For example, the transcriptional terminator isolated from the crystal protein of B. thuringienesis.

The resulting constructions may be inserted into a suitable, cellulose-producing microorganism and either replicated independently using an appropriate expression vector or, if plasmid instability is thought to be a problem, the promoter-gene construct may be integrated directly into the chromosome of the host microorganism. The cellulose-producing host microorganism may be either a cellulose synthase negative strain or a cellulose synthase positive strain. In the former example, one or more of the genes of the cellulose synthase operon will restore the cellulose producing ability of the host microorganism. It is expected in the latter example that the introduction of the recombinant cellulose synthase operon will increase both the cellulose synthase activity and cellulose production of the recombinant strain.

The nucleotide sequence encoding the genes of the bacterial cellulose synthase operon of the invention may be expressed in a variety of procaryotic systems, including E. coli, Streptomyces, Acetobacter, Agrobacteria, Rhizobium, Pseudomonas, Alcaligenes, Zymomonas, Zoogloea, blue-green algae, and Sarcina ventriculi, with E. coli and Acetobacter being preferred.

Since cellulose synthase is of bacterial origin, vectors suitable for the expression of cellulose synthase are known in the art and may include hybrid shuttle vectors for the development of host-vector systems for acetic acid bacteria, such as Acetobacter. Fukaya, et al., (1985) Agric. Biol. Chem. 49:2083-2090, describe several shuttle vectors of relatively small size, with selectable antibiotic gene markers, and capable of replicating in E. coli and Acetobacter. Fukaya, et al., (1989) App. Env. Microbiol. 55:171-176, describe a shuttle vector for E coli and Acetobacter species, pmv24, which allows translation of a cloned sequence as a fusion protein with β-galactosidase.

The present invention also provides an endogenous Acetobacter vector for use in the cloning and expression of the cellulose synthase genes. This vector, called p824, is small, and lacks the large mobilization region present on the pKT230cos5 conjugation vector described herein. The small size of p824 makes it easier to manipulate as a cloning vector. Analysis of the insert DNA in this vector should also be greatly facilitated due to its small size. The p824 vector can be used to directly clone genes from one Acetobacter host to another, thereby eliminating the host restriction barrier and associated rearrangements/deletions that occur during conjugation of pKT230cos5 cosmids from E. coli to Acetobacter.

The endogenous plasmid may be used for direct Acetobacter-Acetobacter transfer and may also be used to develop shuttle vectors. Thus, the present invention also provides both cloning and expression vectors (for E. coli and Acetobacter species), using appropriate control sequences which allow direct transcription and translation of a desired sequence using, for example, the E. coli lac promoter and its translational initiation signal. Transformation of Acetobacter with these vectors results in transformation efficiencies useful for various types of cloning and expression experiments.

## Introduction of DNA into Acetobacter

### A. Conjugations

Conjugation is a technique useful for transferring foreign DNA into a targeted host cell. The cosmid vectors constructed in the present invention are not easily transferred from E. coli for replication in a different bacterial host such as Acetobacter. In such cases, a helper plasmid, such as pRK2013 (Figurski and Helinski, (1979) Proc. Natl. Acad. Sci. USA) 76:1648-1652) may be used to assist in transferring the cosmid vector containing the desired DNA insert from the donor cells into the recipient cells. For conjugation, donor, mobilizing and recipient cells are mixed and mated on plates. Cells which acquire the donor are selected for by growth on plates containing antibiotic to which the transformed recipient cells are resistant. Miller, supra, also discloses a conjugation procedure where nutritional markers are used to select recipient cells. Another conjugation procedure suitable for mass screening is the "multiple spot conjugation method" set forth in detail in Example V, infra.

B. Transformations

Two basic parameters affect the efficiency of electroporation. One is the field force and the other is the time period (pulse duration) over which the field force decays.

The optimal field force for the Acetobacter strains used in the present invention varies between 9 and 9.5 5 KV/cm. A study of pulse duration (RC values) using from 25-100 uF (series) versus 200-1200 ohms demonstrated that 25 uF/750 ohms (=18.75 msec) is optimal for Acetobacter strain 1306-24. The level of transformation obtained (approximately $10^7$ transformants/ug) is about $10^2$-fold higher than that obtained by chemical treatment (Fukaya, et al., (1985) Agri. Biol. Chem. 49:2091-2097).

It was also determined that the transformation frequency of DNA prepared from Acetobacter is $10^3$-fold higher than when the DNA is prepared from E. coli.

In order that the invention described herein may be more fully understood, the following examples are set for illustrate purposes only and are not to be construed as limiting the scope of this invention in any manner.

Example I

Preparation of Cellulose Negative Acetobacter Strains

In the following examples a number of culture media were used. Unless otherwise indicated the media were formulated as follows:

R20-2 medium has the following composition:

| Compound | Final Concentration |
|---|---|
| Bacto-peptone | 5 g/l |
| Yeast Extract | 5 g/l |
| $Na_2HP0_4$ | 5 g/l |
| Citric Acid | 1.15 g/l |
| Carbon Source | As specified (if not specified, 2% glucose) Final pH 5.0 +/- 0.2 |

Minimal medium R70 (also referred to as Acetobacter Minimal Medium or "AMM"), has the following composition:

| Compound | Final Concentration (mM) |
|---|---|
| $(NH_4)_2SO_4$ | 25 |
| $KH_2PO_4$ | 7.3 |
| $MgSO_4$ | 1.0 |
| $FeSO_4$ | 0.013 |
| $CaCl_2$ | 0.10 |
| $Na_2MoO_4$ | 0.001 |
| $ZnSO_4$ | 0.006 |
| $MnSO_4$ | 0.006 |
| $CuSO_4$ | 0.0002 |
| | pH - 5.0 |
| Glucose | 2% or 4% (w/v) unless otherwise specified |

For all studies using R-70 medium and modifications thereof, the following vitamin mixture was added to the minimal medium at a 100-fold dilution:

| Compound | Vitamin Mixture (mg/L) |
|---|---|
| Inositol | 200 |
| Niacin | 40 |
| Pyridoxine HC1 | 40 |
| Thiamine HC1 | 40 |
| Ca Pantothenate | 40 |

(continued)

| Compound | Vitamin Mixture (mg/L) |
|---|---|
| Riboflavin | 20 |
| Para-aminobenzoic acid | 20 |
| Folic Acid | 0.2 |
| Biotin | 0.2 |

R70-2 medium is a modified form of R70. R70-2 had the following composition:

| Ingredient | Final Concentration (mM) |
|---|---|
| $(NH_4)_2SO_4$ | 25 |
| $KH_2PO_4$ | 7.3 |
| Na Citrate | 4.0 |
| $MgSO_4$ | 1.0 |
| $FeCl_3$ | 0.01 |
| $CaCl_2$ | 0.10 |
| $Na_2MoO_4$ | 0.001 |
| $ZnSO_4$ | 0.005 |
| $MnSo_4$ | 0.001 |
| $CuSo_4$ | 0.001 |
| $CoCl_2$ | 0.001 |
| $NiCl_2$ | 0.001 |

| Ingredient | Final Concentration (mM) |
|---|---|
| vitamin mixture | 10 ml/liter |
| Glucose | as specified (usually 2 or 4%, w/v) |
| final pH = 5.09 ± 0.2 | |

Production of Mutant Acetobacter Strains

Three tissue culture flasks containing 100 ml R70 + 2% glucose, 2% corn steep liquor E804E (CSL, Corn Products, NJ) medium were inoculated with Acetobacter strain 1306-21 (ATCC No. 53524) (one frozen 2 ml vial to each flask). The flasks were incubated statically for 23 hr at 30°C.

The pellicles formed by the Acetobacter cells in culture were ascetically removed with forceps, blended for approximately 15 seconds and filtered through 4 layers of sterile cheesecloth. The cells were washed two times with 0.9% NaC1 by centrifugation for 10 min at 7500 rpm, at 4°C. The cells were resuspended in 20 ml 0.9% NaCl and filtered once more through 4 layers of cheesecloth to remove any remaining clumps.

Mutagenic conditions were selected to give a cell kill of 95% to 99.9%. The cultures were incubated at 30°C. The mutagen was ethyl methansulfonate (EMS, Sigma, St. Louis, MO). The EMS concentrations ranged from 1% to 2% (v/v) and the incubation times ranged from 60 min to 210 min. Similar conditions were used with Acetobacter strains 1306-3 and 1306-11 (ATCC Nos. 53264 and 53263, respectively).

Two procedures were used to isolate the $Cel^-$ Acetobacter strains, 1) mutagenesis without expression, and 2) mutagenesis with expression.

Mutant Acetobacter Strain from EMS Mutagenesis Without Expression

Acetobacter strain 1306-21 was treated with EMS and then directly plated on R20-2 medium to determine percent survival. The plates from the EMS mutagenesis of 1306-21 were examined for potential $Cel^-$ colonies as follows. The mutagenized culture was plated and after seven (7) days presumptive $Cel^-$ colonies were picked. $Cel^-$ mutants may be identified on plates as flat and shiny colonies whereas wild type colonies have a rough, dry appearance. In agitated culture $Cel^+$ strains form pellets while $Cel^-$ cultures produce a suspension of single cells. The frequency of $Cel^-$ colonies was determined to be in the range of from 0.05% to 2.0%. A similar technique was used to isolate $Cel^-$ strains from Acetobacter strains 1306-3 and 1306-11 and similar mutation frequencies were observed.

Mutant Acetobacter Strains from EMS Mutagenesis with Expression

Samples (approximately 0.05 ml) of EMS-treated Acetobacter cells (strain 1306-21) were inoculated into tubes (2 ml, R20-2 broth) and allowed to grow as standing cultures, to permit expression of mutated genes. Culture samples were serially diluted and plated on R20-2 plates. After incubation, the colonies were screened for potential $Cel^-$ types using the above-described protocol and similar mutation frequencies were obtained. A similar technique was used to isolate $Cel^-$ strains from Acetobacter strain 1306-3 and 1306-11.

Example II

Characterization of Cellulose Negative Acetobacter Strains Cellulose Synthase Activity Assay

The cellulose synthase assay used to detect in vitro cellulose synthase activity of $Cel^-$ Acetobacter mutant strains was a modification of the procedure described by Aloni, et al., (1982) Proc. Natl. Acad. Sci. USA 79:6448-6452, which measures production of alkali insoluble polymers (cellulose) from uridine 5'-diphospho-($^{14}C$)-glucose (UDPG). This assay was adapted for the screening of Acetobacter $Cel^-$ mutants. After incubation (10 min. at 30°C), unreacted UDPG in each reaction mixture was separated from the cellulose by heating with NaOH (95°C for 1 hr) and filtering. The radioactively-labeled ($^{14}C$) cellulose retained on the filter was then quantitated by scintillation counting.

The amount of total protein used in the assay depends upon the state of purification of the cellulose synthase. Two or three different sample dilutions were assayed to obtain at least one result in the linear range of the assay (<20% of total UDPG consumed).

The 0.2 ml assay mixture contained, as final concentrations, 0.2mM ($^{14}C$) UDPG (7.5 cpm/pmole), 50 mM Tris, pH 7.5, 10 mM $MgC1_2$, 1 mM EDTA, 2 mM $CaC1_2$, and the sample to be assayed. Cyclic diguanosine monophosphate (c-di-GMP) was added to 5 uM to some of the assay tubes (c-di-GMP activates the cellulose synthase). Controls included a tube containing no added sample, and a tube containing the sample, denatured prior to the incubation by adding 4 ml of 0.5 M naOH.

The timed reactions were commenced by adding enzyme, vortexing the mixture, and placing the tubes into a 30°C water-bath. Succeeding tubes were started at timed intervals. After 10 min, each reaction was terminated by removing the tube from the water-bath, adding 4 ml of 0.5 M NaOH, and vortexing. When all the reactions were stopped, about 20 mg cellulose was added to each tube to act as carrier for the ($^{14}C$) cellulose. The tubes were then heated in a water bath 95°C for one hour to digest the cells.

Using a vacuum manifold, the contents of each assay tube were filtered through a Whatman GF/A filter to isolate the cellulose product (removing any unreacted $^{14}C$ UDPG), by passing the reaction mixture through the filter; rinsing the assay tube 3X with deionized water, passing the rinse water; washing the filter 2X with 20 ml deionized water; then with 20 ml 0.5 M HC1; followed by 20 ml deionized water and 20 ml methanol.

Cellulose production was quantified by scintillation counting. Each filter was placed in a scintillation vial with 10 ml scintillation fluid (NEN Atomlight, Boston, MA) and quantitative cellulose production determined by ($^{14}C$) UDPG incorporation into base-insoluble material by scintillation counting. To obtain the specific activity of the ($^{14}C$) UDPG, an (aliquot of the 2 mM) ($^{14}C$) UDPG stock solution was counted.

The total possible cpm of the assay was determined from the stock-solution aliquot, and the fraction of total UDPG consumed from each assay tube was calculated as follows:

$$\text{Fraction of total UDPG consumed} = \frac{((\text{cpm filter})-(\text{cpm of blank}))}{(\text{total counts})-(\text{cpm of blank})}$$

The nmole of UDPG consumed per minute was calculated as:

$$\text{nmole UDPG/min} = (\text{fraction of total UDPG consumed}) \text{ X } (40{,}000 \text{ pmole}) \text{ X } (1/20 \text{ min})$$

Activity was expressed on the basis of nmole per minute per ml sample, or nmole per minute per mg protein.

Assay for Diguanylate Cyclase Activity

The diguanylate cyclase assay was used to identify Acetobacter hosts lacking two or more specific activities, such as a deficiency in cellulose synthase activity and diguanylate cyclase activity. The enzyme diguanylate cyclase catalyzes the production of c-di-GMP, a cellulose synthase activator, from guanosine triphosphate (GTP).

The cells were grown, washed, and sonicated as described below for the screening assay. Diguanylate cyclase activity was measured using an assay similar to that reported in Ross, et al., (1987) Nature 325:279-281. Sonicated cells (1 mg/assay tube) were incubated for 10 min at 30°C in a 0.1 ml reaction mixture containing 0.2mM [alpha$^{32}$P] GTP, 50 mM Tris HCl, pH 7.5, 10 mM $MgC1_2$, 1 mM EDTA, 5 ml $CaC1_2$, 2 mM phosphocreatine, and 24 units/ml creatine phosphokinase. The reaction was terminated with 10 ml (w/v) 100% trichloroacetic acid. After centrifugation to remove the precipitate, 10 ml of each reaction mixture was spotted on a thin layer chromatography (TLC) plate (Polygram Cel 300 PEI, Macherey-Nagel, Duren, W. Germany, U.S. distributor Sybron/Brinkman, Westbury, NY). The TLC plate was developed in 1.5 M $KH_2PO_4$, pH 3.65, for about 2 hr, autoradiographed, and the areas on the TLC plate corresponding to GTP an cylic-di-GMP were excised and counted in a scintillation counter.

Screening Assay

To observe restoration of cellulose synthase activity, one needs to isolate a strain which is cellulose synthase negative and diguanylate cyclase positive. The following screening assay is a modification of the cellulose synthase activity assay described above and may be used to determine mutations involving two different enzymes by comparing cellulose synthase activity in the presence or absence of GTP or c-di-GMP. The assay measures cellulose production by suspended, sonicated cells.

Mutant Acetobacter strains were classified by assaying sonicated cells under several different conditions. Three classes of Acetobacter mutations were identified. Class I (cellulose synthase negative) strains produced no cellulose under any assay conditions. Class II (diguanylate cyclase negative) strains produced cellulose in the presence of c-di-GMP, but not the in the presence of GTP. Class III mutants produced cellulose following activation by either GTP or c-di-GMP. Mutants in Classes II and III contain cellulose synthase, and show activity in vitro, but produce little or no cellulose in vivo. The deficiency in the Class III mutants has not been defined biochemically.

The screening assay was conducted under the following conditions: 1) no nucleotide added; 2) 0.4 mM GTP added; and 3) 5 uM c-di-GMP added.

Except for the source of enzyme and the buffer used, the assay procedure and controls were as described above for the cellulose synthase activity assay.

Six mutant Acetobacter strains were assayed in a single screening. Each time an Acetobacter strain having both cellulose synthase activity and diguanylate cyclase activity was included to monitor consistency between assays.

The selected Acetobacter strains were grown for approximately 24 hours at 30°C. The growth medium contained R70-2, 1% Ambrex 1003 (TYE, Universal Foods, Milwaukee, WI), 4% fructose, 0.01% v/v Dow Corning Antifoam B, and 50 mM 3,3-di methylglutaric acid (DMG), pH 5.0., 25 ml of medium was inoculated in a 125 ml baffled shake flask with the cells from a frozen seed vial. Before collecting the cells, R70-2 + 0.5% TYE and 1% glucose agar plates were streaked to monitor for contamination. The plates were incubated at 30°C for about 3 days.

Prior to collecting the cells, 5 ml of 0.6 M EDTA was added to the medium to prevent clumping. The cells were centrifuged (5000 rpm, 10 min, JA21 rotor) and the supernatant discarded. The pellet was suspended in 20 ml 50 mM potassium phosphate buffer, pH 6.0, and 5 ml 5 M NaC1 was added to reduce clumping.

Cell density was measured with a Klett meter (10 Klett Units ("KU") = approximately 40 mg cells/ml). The cells were again centrifuged (5 K rpm, 10 min, JA21 rotor) and the supernatant discarded. The pellets were suspended to 20 mg cells/ml in 50 mM N-(2-hydroxyethyl) piperazine, N'-3- propanesulfonic acid (EPPS, Sigma) buffer, pH 7.5.

To sonicate the cells, 0.5 ml of each cell suspension was transferred into a 1.5 ml Eppendorf centrifuge tube. Each tube was sonicated in a cup sonicator (Branson Sonic Power Co., Model 350, Plainview, NY) for 1 min., 80% duty cycle, setting of 7. The assay was performed as described above using 50 UL of sonicated cell suspension for each assay tube, substituting 50 mM EPPS, pH 9.0 for the 50 mM Tris, dropping the 1 mM EDTA, and adding 20 mM $MgCl_2$ in place of 10 mM $MgCl_2$. Each assay condition was duplicated and the duplicates were averaged in the calculations.

Several Cel$^-$ strains were biochemically characterized. Strain 1306-24 (derived from 1306-3) was found to have normal diguanylate cyclase activity but was defective in cellulose synthase activity. Strains 1306-42 (derived from 1306-21) and C90-1 (derived from 1306-21) were defective in both diguanylate cyclase and cellulose synthase activities. These strains were picked for subsequent studies.

Example III

Construction of Cosmid Vector and Conjugation Procedure

A new cosmid vector, pKT230cos5, was constructed as summarized in Figure 2 for the cloning of a cellulose synthase gene. This vector contains a streptomycin resistance gene (Sm), the cos fragment of phage lamdga and cloning sites for insertion of foreign DNA.

A 1.85 kb DNA fragment containing the lambda cos site was excised from plasmid pVK100 (Knauf and Nester, (1982) Plasmid 8:45-55) by digestion with the restriction enzyme Bg1II and was cloned in the BamHI site in plasmid pUC19 (New England Biolabs Catalog). The new plasmid, pUC19cos2, was digested with the restriction enzymes HindIII and XmaI and the HindIII-XmaI fragment containing the 1.85 kb cos-containing fragment was cloned into XmaI- and HindIII-digested plasmid pKT230, thereby inactivating the kanamycin-resistance gene. The resulting vector, cosmid pKT230cos5, is not self-transmissible from E. coli to Acetobacter. Therefore, a helper plasmid, pRK2013 is necessary to mobilize the transfer of PKT230cos5 from the donor cells into the recipients cells of Acetobacter strain 1306-24. E. coli strain MM294 transformed with pKT230cos5 was grown at 37°C in R2 medium (20.0 g Tryptone, 10.0 g yeast extract, 10.0 g NaCl, 1.0 L distilled $H_2O$, pH 6.9 plus g/l glucose (R2-4) and optionally, 15 g/L Bacto agar) containing 100 ug/ml Sm to a Klett reading of 150 KU. E. coli strain HB101 containing the mobilizing plasmid pRK2013 was grown at 37°C in R2-4 medium containing 50 ug/ml Km to a Klett reading of 150 KU. The Acetobacter recipient cells 1306-24 were grown at 30°C in R20-2 medium to a Klett reading of 200 KU. 1 ml of each donor, mobilizing and 2 ml recipient cells were mixed and filtered through a 0.2 micron Gelman disposable filter (Gelman, Ann Arbor, MI), washed twice with 10 ml of R2 medium without antibiotics. The filter was placed on agar plates containing R2-4 medium. The plates were incubated at 30°C for 3 hr to allow mating to take place. After mating between the three strains, the conjugation mixture was resuspended in 2 ml of 0.9% sodium chloride. 0.1 ml of this solution was plated on R20-2 medium agar plates containing 50 ug/ml Sm and 20 ug/ml Cm. The plates were incubated at 30°C for 5 days. Acetobacter strain 1306-24 is naturally resistant to 20 ug/ml Cm, while E. coli strains are sensitive to it. Therefore only Acetobacter colonies which had acquired the donor plasmid grew on these selection plates. Subsequent restriction analysis showed that the cosmid pKT203cos5 did not undergo any rearrangements in Acetobacter strain 1306-24.

Example IV

Construction of an Acetobacter DNA Library

Lambda phage will package DNA from 38 to 52 kb in length if cos sites are present at both ends. Since the vector pKT230cos5 was relatively small (13.5 kb), a large amount of Acetobacter DNA (28 to 37 kb) could be inserted and packaged in lambda phage particles. Presuming the genome size of Acetobacter is equivalent to E. coli, only 700 to 1000 clones are presumed necessary to have a complete gene bank. DNA banks were constructed from Acetobacter strain 1306-3 as follows.

About 26 mg of nucleic acid were isolated from lawns of Acetobacter 1306-3 on R20-2 agar plates. This nucleic acid was treated with RNase A and RNase T1 to degrade the RNA in the sample. A total of 560 ug of DNA was recovered. This DNA was partially digested with the restriction enzyme Sau3A at four different enzyme concentrations. The DNA was fractionated by size on a 10-40% sucrose gradient. The fraction containing the largest number of DNA molecules between 27-38 kb was selected (approximately 2 ug of DNA).

Approximately 1 ug of this DNA was ligated into BamHI-cleaved and dephosphorylated pKT230cos5 DNA, and the ligation mixture was packaged into lambda phage particles. The phage particles were then used to infect E coli strain K802 recA-. Cosmid DNA, isolated from six random E coli isolates, was used to determine the sizes of the inserted DNA fragments. The six clones had DNA inserts ranging from 8 ^To 40 kb with an average size of 28 kb. Approximately 2000 clones were picked, individually grown in microliter dishes, and stored for later screening. The bank was designated pKT230cos5:1306-3A2.

Example V

Identification and Isolation of Cloned DNA that Restored Cellulose Synthase Activity in Cellulose Negative Acetobacter Strains

A genetic complementation assay for restoration of cellulose synthase activity was used to isolate cosmid DNA capable of restoring cellulose synthase activity in the Cel- Acetobacter mutants.

Screening of Acetobacter Gene Bank Through Conjugations

Cosmids from the gene bank pKT230cos5:1306-3A2 (obtained as described in Example IV) were transferred to recipient mutant Acetobacter strain 1306-24 for screening using the mobilizing plasmid pRK2013 in a multiple spot conjugation method described below.

Acetobacter strain 1306-24 was grown in R20-2 medium at 30°C with shaking for approximately 28 hr to a Klett reading of 100-150 KU. E. coli HB101/pRK2013 was grown in R2-4 medium and 50 ug/ml kanamycin (Km) at 37°C with shaking to a Klett reading of 100 to 150 KU. E. coli K802 recA- (pKT230cos45:13060-3A2) cultures were inoculated from frozen microliter trays with a flamed frog into microliter dishes containing 100 ul R2-4 medium plus 50 ug/ml Sm and incubated at 37°C without shaking for approximately 18 hr. The E. coli HB101/RK2013 culture was centrifuged down, washed with an equal volume of 0.9% saline to remove the Km, centrifuged down again and concentrated by resuspending in one tenth the original volume of saline. A 10 ul volume of this concentrated E. coli HB101/pRK2013 was added to each microliter well containing 100 ul of 18 hr E. coli K802 recA- pKT230cos5:1306-3-A2 culture. 10 ul volumes of the mixed HB101/pRK2013 and K802 recA- pKT230cos5:1306-3-A2 cultures were spotted onto dry 150 mm R2-4 plates using an 8-channel pipette and in an array corresponding to the pattern of microliter wells so that the donor of each transconjugant could be traced back to its location in the E. coli cosmid bank. Once the spots were dry, an additional 10 ul of each mixed culture was layered over the original spot and allowed to dry. This was repeated until 50 ul of each mixed culture was laid down. The Acetobacter 1306-24 culture was spun down and resuspended in one tenth the original volume of 0.9% saline. A 10 ul amount of this concentrated culture was layered over each HB101/pRK2013 and E. coli K802 recA- pKT230cos5:1306-3A2 spot on the R2-4 plates with the 8-channel pipette. The spots were allowed to dry, then the conjugation mixtures were incubated at 30°C on the R2-4 plate for 3 hr.

To determine which transconjugants produced cellulose, each of the mating mixtures was scraped from the mating plates with a sterile toothpick and inoculated directly into 2 ml R70-2 containing 0.5% TYE, 3% glucose, 25 mM DMG, 20 ug/ml Cm and 50 ug/ml Sm in 13 x 100 mm tubes ("test tube selection screen"). The antibiotic Cm inhibits the growth of the E. coli donor and helper parents. The antibiotic Sm inhibits the growth of the Acetobacter strain 1306-24 which had not received a cosmid. The tubes were incubated at 30°C and checked for formation of pellicles at day 7 and day 14. This procedure allowed visualization of any cellulose that the transconjugants produced.

Using the multiple spot conjugation method described above for cosmid transfer and the test tube selection screen for cellulose production, 487 cosmids from the bank pKT230cos5:1306-3A2 were used in matings with the Acetobacter cellulose synthase mutant 1306-24. Of these conjugations 386 showed successful cosmid transfer. Three of these matings mixtures formed a pellicle in the standing test tube screen after 14 days incubation at 30°C. These three cosmid donor cultures were designated T19G9, T20A1, and T20B6.

None of the transconjugant colonies arising when these conjugation mixtures were directly plated, showed a $Cel^+$ phenotype. It is only when conjugation mixtures were inoculated into standing test tubes that cellulose production would be detected. In later studies it was observed that restoration of $Cel^+$ activity was due to recombination and not to true complementation. This might explain the need for a screen using the test tube selection, rather than relying solely on a plating protocol.

Confirmation of Restoration of Cellulose Synthase Activity in Filter Matings

Cosmids T19G9, T20A1 and T20B6 whose 1306-24 transconjugants produced a pellicle in the test tube selection screen described above were further tested in transfers done by the filter conjugation method with Acetobacter 1306-24 (recipient), E. coli HB101pRK2013 (helper plasmid stain), and -E. coli pKT203cos5:1306-3A2 T19G9, -T20A1, and -T20B6 (donor strains) according to the procedure in Example III.

100 ul of the mating mixtures were inoculated for a test tube selection screen. Conjugation frequency for these conjugations was determined by plating serial dilutions of the mating mixtures directly after conjugation on R20-2 plates containing 20 ug/ml Cm and 40 ug/ml Sm. As a negative control the vector pKT203cos5 as transferred to 1306-24 in parallel with these conjugations, plated and inoculated into test tubes. These 1306-24 pKT230cos5 transconjugants formed no pellicles in the standing test tube screen and served as a control to distinguish between Cel growth and pellicle formation in standing test tubes. Of the three cosmids previously identified, only cosmid pKT230cos5 1306-3A2 T19G9 gave positive results, i.e., converted strain 1306-24 from $Cel^-$ to $Cel^+$ in standing test tube screen. The frequency of conjugation of the cosmid T19G9 was $7.0 \times 10^{-8}$ per recipient cells.

## Example VI

## Construction of Cosmids Carrying Truncated And Full-Length Cellulose Synthase Gene B

Conjugation between the E. coli carrying pKT230cos5T19G9 and Acetobacter 1306-24 was performed on a filter. 100 ul of the conjugation mixture was inoculated for a test tube selection screen as described above. After seven days a pellicle formed in the test tube and was blended in a blender with 25 ml of 0.9% NaC1. The resulting suspension was streaked for single colonies on an R20-2 plate containing 20 ug.ml Cm and 40 ug/ml Sm. A $Cel^+$ colony was picked and designated 1306-24 T19G9#106. The cosmid isolated from 1306-24 T19G9#106 was 16.6 kb in length. When E. coli strains K802 recA- carrying T19G9#106 were used as the cosmid donors in a conjugation with 1306-24 as the recipient, Acetobacter transconjugants showed a $Cel^+$ phenotype in 100% of the test tubes in the test selection screen. Therefore, the 16.6 kb cosmids were able to restore cellulose synthase activity in the $Cel^-$ mutant of 1306-24. Restriction analysis indicted that the T19G9#106 was a deletion product of T19G9. Nucleotide sequence analysis after the intact gene was cloned confirmed that cosmid T19G9#106 was carrying a truncated cellulose synthase B gene wherein the deletion site was located 142 bp 3' from the unique BamHi site in the coding region of the gene.

In a Southern hybridization, a 1.8 kb BamHI-SmaI fragment from cosmid T19G9 strongly hybridized with the oligo probe MK172 (its sequence spans from nucleotide 4564 to 4582 in Figure 1). If the molecular weight of the cellulose synthase was not greater than 120 kd, the 1.8 kb BamHi-Sma-I fragment should contain the 3'-end of the gene. To confirm this hypothesis, a plasmid carrying a full-length cellulose synthase gene was constructed in a three piece ligation as follows.

The cosmid vector pKT230cos5 was first digested with BamHI an the ends were repaired with the Klenow enzyme in the presence of all four deoxyribonucleotide triphosphates. The DNA was further digested with HindIII. A 3.5 kb BamHI-SmaI fragment from T19G9 containing the 3'-end of the cellulose synthase gene and the HindIII-BamHI fragment from T19G9#106 carrying the 5'end of the cellulose synthase gene were ligated into the HindIII-BamHI repaired vector pKT230cos5 to construct TRT18-1. The construction for TRT18-1 is shown in Figure 3.

## Example VII

### In Vitro Cellulose Synthase Activity In Cellulose Positive Transconjugants

A 3 kb region of Acetobacter genomic DNA (cosmid T19G9#106) could restore a $Cel^+$ phenotype to Acetobacter strain 1306-24 (Example VI). The present example describes the recovery of cellulose synthase activity in the transconjugant Acetobacter strain 1306-24. The assay used was described as the screening assay in Example II.

Controls consisted of Acetobacter strain 1306-3 ($Cel^+$), and strain 1036-24 ($Cel^-$) each carrying the vector pKT230cos5. The $Cel^+$ strain 1306-24 T19G9#106 ("the transconjugant") carried a 3kb fragment of Acetobacter geonoic DNA in the vector pKT230cos5. The cells were grown as described in Example II, with addition of 50 mg/l Sm, and 20 mg/l Cm. The cells were collected, separated from cellulose, washed, brought to concentration and sonicated as previously described. In vitro cellulose synthase activity was measured and a BCA (bicinchoninic acid) protein assay developed by Pierce (Chemical company, Rockford, IL) was used to measure the protein concentration of the sonicated cell preparations. Strains were assayed at a concentration of 2 mg cells/ml. Strains 1306-24 pKT230cos5 and 1306-24 T19G9#106 were also measured at 5 mg cells/ml to check for a possible concentration effect.

As shown in Table 1, the cellulose synthase activities of strains 1306-24 pKT230cos5 and the transconjugant 1306-24 were not affected by the cell concentration of the assay. The activity of 1306-3 pKT230cos5 was similar to the activity of strain 1306-21 (not shown). Thus the vector pKT230cos5 does not appear to affect the cellulose synthase activity. Strain 1306-24 demonstrated a low level of cellulose synthase activity upon activation by c-di-GMP. This was true for most Class I (cellulose synthase negative) Acetobacter mutants. Although the mutation in cellulose synthase activity appears somewhat leaky in vitro, the cells appear to make very little cellulose in vivo (in shake flasks). The 3 kb insert increased the cellulose synthase activity of strain 1306-24 roughly ten-fold (0.4 to 4.1 nmole/min mg) at 5 mg cells/ml in the assay tube. This correlated with the appearance of the cellulose positive phenotype. The transconjugant strain 1306-24 had the same c-di-GMP-stimulated activity as did the cellulose positive strain 1306-3.

Results for TRT18-1, determined in a separate experiment using appropriate controls, confirmed that this plasmid was able to convert a $Cel^-$ phenotype to $Cel^+$, and secondly, that, in the in vitro assay, the activity of the enzyme was comparable to that observed for the pKT230cos5 vectors carrying the 3 kb T19G9 #106 Acetobacter DNA insert shown in Table 1.

Table 1

| Conversion of Strain 1306-24 from $Cel^-$ to $Cel^+$ Recovery of In Vitro Cellulose Synthase Activity | | | |
|---|---|---|---|
| Strain | Cell Concentration in assay | Cellulose Synthase Activity (nmole UDPG consumed) (min-mg) | |
| | (by Klett units mg/ml) | No c-di-GMP + c-di-GMP | |
| 1306-3pKT230cos5 | 2 | 0 | 4.1 |
| 1306-24pKT230cos5 | 2 | 0.2 | 0.6 |
| 1306-24+insert[1] | 2 | 0.2 | 3.9 |
| 1306-24pKT230cos5 | 5 | 0.1 | 0.4 |
| 1306-24+insert[1] | 5 | 0.1 | 4.1 |

[1] pKT230cos5 with the 3 kb T19G9#106 Acetobacter DNA insert.

Example X

Cloning of a 3.5 kb Endogenous Plasmid From Acetobacter Strain

Acetobacter CMCC824, a proprietary strain of Cetus Corporation, a cellulose producing strain isolated from a vinegar culture (CMCC824) contains a 3.5 kb plasmid. To isolate this plasmid, a single colony from an R20-2 plate was inoculated into 50 ml of R20-2 and grown without shaking at 30°C for 48 hours. The pellicle was washed twice in 100 ml of 50 mM Tris, 10 mM EDTA, pH 8.0, homogenized in a Beckman blender and filtered through cheesecloth. The cells were pelleted and plasmid DNA was prepared following a modified SDS lysis procedure by Guerry, et al., (1973) J. Bacteriol 116:1064-1066. Of the restriction enzymes tested, only Sac1 was able to linearize this plasmid.

SacI-digested plasmid was ligated to SacI-digested plasmid pUC18 (New England Biolabs) for cloning in E. coli. This shuttle vector was designated pUC18-824. In order to physically map pUC18-824, the plasmid was digested with a variety of restriction endonucleases. A restriction map of pUC18-824 is shown in Figure 4.

Example XII

Construction of pUC18-824 Containing The Cellulose Synthase B Gene

The cellulose synthase B gene can be isolated from plasmid TRT181, the construction of which is taught at Example VI, or alternatively, since the nucleotide sequence of this gene is provided herein, the full-length gene may be directly synthesized by chemical means or may be obtained from the constructed gene bank using a primer. For example, the primer oligonucleotide MK170 (TGCCCTGGCCAGATGTCAGCA) was used to probe the 1760 individual cultures from the constructed gene bank, and six clones were isolated for further characterization. Three cosmids isolated from three of these clones were designated as T5A1, TIC2 and T5D2.

The 8.3 kb SmaI fragment carrying the cellulose synthase B gene and about 3 kb upstream sequence (see the restriction map in Figure 5) was cloned into the SmaI site of pUC18-824. The resultant plasmid carrying the opposite orientations of the 8.3 kb SmaI restriction fragment, were designated as pUC18-824 FS-1 and pUC18-824 FS-6, respectively. A restriction map of FS-6 is provided in Figure 6. When such plasmid were transformed into 1306-24 (a cellulose synthase deficient strain), 1306-42 and C90-1 (both deficient in diguanylate cyclase and cellulose synthase activities), the transformants showed the $Cel^+$ colony phenotype on plates. Therefore, in contrast to the "recombination events" observed in the earlier experiments, it was concluded that the protein encoded by the 8.3 kb Acetobacter DNA insert is directly capable of complementing the cellulose synthase mutations carried in the mutants.

In vitro assays confirm the ability of the plasmid pUC18-824 FS-1 and pUC18-824 FS-6 to restore cellulose synthase activity to the cellulose synthase negative mutant strain 1306-24. The in vitro cellulose synthase assay of the transformants and control strains was carried out as described in Example VII. As shown in Table 3, the transformants, 1306-24 pUC18-825 FS-1 and 1306-24 pUC18-824 FS-6 showed cellulose synthase specific activities higher than that of the original $Cel^+$ parent strain, 1306-3 (1.3X and 1.8X, respectively).

Table 3

| Conversion of Strain 1306-24 from $Cel^-$ to $Cel^+$: Recovery of In Vitro Cellulose Synthase Activity | | |
|---|---|---|
| Strain | Cellulose Synthase Activity nmole UDPG incorporated/(min-mg protein) | |
| | no c-di-GMP | + c-di-GMP |
| 1306-3 | 0.04 | 2.05 |
| 1306-24 | 0.06 | 0.07 |
| 1306-24 pUC18-824 | 0.06 | 0.10 |
| 1306-24 pUC18-824 FS-1 | 0.10 | 2.59 |
| 1306-24 pUC18-824 FS-6 | 0.10 | 3.71 |

Example XIII

Construction of an Expression Vector in Acetobacter

Plasmid pUC19 (New England Biolabs) was digested with the restriction enzyme SacI. The linearized plasmid was ligated with SacI-restricted Acetobacter plasmid 824. The resultant plasmid was designated pUC19-824. The cellulose synthase gene from the 8.3 kb SmaI fragment was cloned as a HindIII-SmaI fragment (i.e., 4.9 kb) into the HindIII-SmaI sites in the linker region of the pUC19 plasmid (see Figure 8) so that its transcriptional direction was identical to the lac promoter. This construction placed the gene under the control of the lac promoter in Acetobacter. The plasmid was designated pAL1.

Plasmid pAL1 was used to transform Acetobacter strain 1306-24 and was shown to complement the cellulose synthase-deficient phenotype, resulting in a $Cel^+$ phenotype on plates.

Example XIV

Identification of the cellulose synthase operon

A. Localization of the transcription initiation site

Primer extension: The oligodeoxyribonucleotide G E 1 3 (5'-TGCGGCGATAAGTGCACA-3') was labeled with gamma-$^{32}P$ ATP and T4 polynucleotide kinase. The unincorporated nucleotide was removed by ethanol precipitation. The labeled oligodeoxyribonucleotide was resuspended into 100ul of 0.3M NaOAc solution. The specific activity of the primer was approximately 4 x $10^6$ cpm/pmole. Labeled primer (0.02 and 0.2 pmoles) was used for primer extension analysis.

The primer extension analysis indicated that the transcriptional initiation site was located within the region 5' of the first gene in the cellulose synthase operon. The transcriptional initiation site of the operon is marked by a downward arrow positioned over nucleotide 235 in Figure 1.

B. Cloning of Genes C and D

The construction of pABCD is outlined in Figure 8.

C. Sequence and Structure of the Cellulose Synthase Operon

The nucleotide sequence of the region from pABCD is shown in Figure 1. The cellulose synthase operon is 9217 bp in length and consists of four genes. Genes A, B, C, and D are 2,262 bp, 2,406 bp, 3,957 bp, and 468 bp in length, respectively. The molecular weights determined by and suggested roles of the gene products are as follows:

| Gene Product | Amino Acid Residues | Molecular Weight | Associated Function |
|---|---|---|---|
| A | 754 | 84 kd | Cellulose synthesis in vivo ; diguanylate cyclase and cellulose synthase in vitro activities |
| B | 802 | 85 kd | Cellulose synthesis in vivo; cellulose synthase in vitro activity |
| C | 1319 | 141 kd | Cellulose synthesis in vivo |
| D | 156 | 17 kd | Cellulose synthesis in vivo |

Computer analysis of the DNA sequences at the 3' end of the D gene revealed a region with the potential of forming a stable stem-and-loop structure. As shown in Figure 1 by the underlined section, this region is positioned 26 bp 3' of the termination codon of the D gene and corresponds to a transcription terminator region of the operon.

Example XV

Cell Growth, Cellulose Production and Cellulose Synthase Activity in Recombinant Strains

A. Studies With 1306-21 pUC18-824 pABCD

In this study, overexpression of cellulose synthase activity in 1306-21 pABCD was tested in shake flask experiments. The construction of 1306-21 pABCD was similar to the construction of 1306-3 pABCD (See Example XIV). Culture medium for all stages of the experiment was R70-2 with 10 uM $FeCl_3$, 1% TYE, 25 mM DMG, and 4% glucose (1306-21) or 4% fructose (1306-3). The seed medium contained 0.1% cellulase. Ampicillin was added at 50 ug/ml to medium used for growing plasmid containing cultures. Medium was dispensed into 125 ml baffled flasks, with 25 ml per flask. Strains 1306-21, 1306-21 pUC18-824 (the host strain plus the shuttle vector), 1306-21 pUC18-824 pABCD (normal -- the phenotype like parent), and 1306-21 pUC18-824 pABCD (spired -- its growth on plates was raised and more tapered than the parent) were individually tested. Each culture was adjusted to 0.72 g/L (turbidity 1.8 $OD_{680}$) using sterile saline. Test flasks were inoculated with 0.2 ml of seed culture (2% v/v inoculum). Six flasks of each strain were inoculated and incubated at 30°C, 125 rpm, 2-inch throw. Flasks were harvested after one, two, and five days. Duplicate flasks were harvested at each time point for cell mass and cellulose measurements. In addition, cultures from the five day flasks were checked for plasmid retention, by observing antibiotic resistance on plates (patch test). Thirty colonies were tested for each strain.

To measure cellulose production and cell concentration, the flask contents of each sample were transferred to a 100ml beaker. The suspension was then macerated for one minute with a large Tekmar probe at 50% full power. After that the suspension was centrifuged at 5,000 rpm for 10 minutes. The supernatant was discarded and the pellet was resuspended in 15 ml saline solution and incubated for 15 minutes with occasional stirring. The sample was again centrifuged and the above wash step repeated.

The pellet from the second wash step was resuspended in 15 ml of 0.1 N of NaOH and incubated at 60°C with mild agitation for 60 minutes. The suspension was centrifuged and the NaOH supernatant was used to analyze cell concentration while the pellet was used to analyze cellulose concentration.

The pellet was resuspended in 15 ml deionized water and left at room temperature for 15 minutes with occasional stirring. Then the sample was centrifuged and the above wash procedure was repeated for a total of three times. After the last centrifugation step, the cellulose precipitate was dried at 60°C overnight in a vacuum oven and then weighed.

The supernatant was neutralized with HCl (approximately 0.05 ml HCl to 0.5 ml sample) and the protein concentration was assayed by the Lowry method. Cell concentration = protein concentration x 1.54.

Cell growth and cellulose production are summarized in Table 4.

Table 4

| Cell Growth and Cellulose Production | | | | | |
|---|---|---|---|---|---|
| | Cellulose g/L | | Cells g/L | | Cellulose/Cells |
| 1306-21 | - | | - | | - |
| | x | s | x | s | x |
| day 1<br>day 2<br>day 5 | 1.78<br>4.58<br>6.99 | ±0.006<br>±0.32<br>±0.16 | 0.51<br>1.16<br>2.92 | ±0.03<br>±0.02<br>±0.15 | 3.52<br>3.95<br>2.40 |
| 1306-21 pUC18-824 | | | | | |
| day 1<br>day 2<br>day 5 | 0.91<br>3.59<br>6.89 | ±0.01<br>±0.21<br>±0.01 | 0.14<br>0.92<br>3.07 | ±0.06<br>±0.08<br>±0.06 | 6.54<br>3.91<br>2.24 |
| 1306-21 pABCD (spired) | | | | | |
| day 1<br>day 2<br>day 5 | 1.16<br>4.78<br>7.59 | ±0.12<br>±0.05<br>±0.07 | 0.14<br>0.72<br>2.59 | ±0.04<br>±0.04<br>±0.04 | 8.42<br>6.65<br>2.93 |
| 1306-21 pABCD (normal) | | | | | |
| day 1<br>day 2<br>day 5 | 1.06<br>4.40<br>7.28 | ±0.06<br>±0.28<br>±0.10 | 0.14<br>0.76<br>2.81 | ±0.01<br>±0.05<br>±0.01 | 7.41<br>5.82<br>2.59 |
| 1306-3 | | | | | |
| day 1<br>day 2<br>day 5 | 1.87<br>2.67<br>3.02 | ±.17<br>±.21<br>±.01 | 1.38<br>2.11<br>2.82 | ±.11<br>±.11<br>±.19 | 1.36±.01<br>1.27±.04<br>0.96±.09 |
| 1306-3 pUC18-824 | | | | | |
| day 1<br>day 2<br>day 5 | 1.56<br>2.39<br>3.88 | ±.01<br>±.04<br>±.01 | 1.26<br>2.25<br>3.70 | ±.11<br>±.42<br>±.17 | 1.24±.10<br>1.08±.18<br>1.05±.05 |

Table 4 (continued)

| Cell Growth and Cellulose Production | | | | | |
|---|---|---|---|---|---|
| | Cellulose g/L | | Cells g/L | | Cellulose/Cells |
| 1306-3 pABCD | | | | | |
| day 1 | 2.92 | ±.11 | 1.20 | ±.04 | 2.44±.18 |
| day 2 | 3.90 | ±.01 | 2.45 | ±.03 | 1.59±.01 |
| day 5 | 3.66 | ±.13 | 2.97 | ±.07 | 1.24±.08 |

Cellulose to cell ratio is an indicator for cellulose specific productivity. In the first two days of growth the cellulose to cell ratio in pABCD 1306-21 strains is significantly higher than those of the control 1306-21 strains. The pABCD (spired strain) seems to produce more cellulose than the pABCD (normal) strains. Both recombinant strains produce more cellulose than the control strains at the end of growth.

In the strains with the ampicillin marker, the plasmid stability of the day 5 culture was shown to be 100%. The retention of the marker does not measure the degree of rearrangement that may occur with the cellulose synthase genes on the plasmid. It is possible that the strains at day 5 are not producing cellulose at the same rate that they did at day 1 and 2. Evidence supporting this hypothesis is seen in the colony phenotypes from day 5 plating. The spired strain of pABCD failed to show the unusual colony morphology on those plates.

Cellulose production with 1306-3 pUC18-824 pABCD was higher than the 1306-3 and 1306-3 pUC19-824 control strains. This was correlated with an increase in the in vitro activity of cellulose synthase (see below). Cell growth with the two strains was similar. Consequently the cellulose to cell ratio with 1306-3 pUC18-824 pABCD was also significantly higher than that of 1306-3 alone.

Table 5

| Strain | Cellulose Synthase Activity nmoles/(min*mg protein) | | |
|---|---|---|---|
| | Day 1 | Day 2 | Day 5 |
| 1306-3 | 2.3 | 0.7 | 0.3 |
| 1306-3 pUC18-824 | 1.2 | 0.6 | 0.3 |
| 1306-3 pUC18-824 pABCD | 3.5 | 2.3 | 0.3 |

The recombinant strain 1306-21 pABCD was also tested in a Chemap fermentor for cell growth and cellulose production. Contrary to that observed in the flasks, the performance of this strain was similar to that of the parent strain. It is believed that instability of the recombinant strain may be responsible for the absence of enhanced cellulose production. Overexpression of the chromosomal cellulose synthase operon genes would eliminate the instability of the recombinant strain.

B. Studies with pUC18-824 FS6 (AB)

Fifteen isolates of 1306-3 (Cel+) carrying pUC18-824 FS6 (which carries the cellulose synthase promoter and genes A and B) were used to screen for overexpression of cellulose synthase activity. The cells were collected, washed, suspended and broken, and the standard in vitro cellulose synthase assay was carried out as described in Example II.

All of the fifteen isolates demonstrated in vitro cellulose synthase activity significantly in excess of that of the control strain (1306-3 pUC18:824). The activities range from 1.5 to 2.4x higher than the control strain. Two of the fifteen strains screened (#302 and #303) were examined at exponential and stationary phases. The conditions were similar to that described in Example XV except that glucose was replaced with fructose. As shown in Table 5, at day 2 the activities of the control strains 1306-3 and 1306-3 pUC18-824 retained 30% and 40% of the day 1 activity, with declining activity over time. At day 2 isolates #302 and #303 showed a decline to 45% and 50% of the day 1 activity, also with declining activity over time. However, these latter strains both showed about 2x higher activity than did 1306-3 at day 1, while the activities were about 3x higher at day 2.

Table 5

| Strain | Cellulose Synthase Activity nmoles/(min*mg protein) | | |
|---|---|---|---|
| | Day 1 | Day 2 | Day 5 |
| 1306-3 | 5.5 | 1.7 | 0.5 |
| 1306-3 pUC18-824 | 5.9 | 2.4 | 0.3 |
| 1306-3 pUC18-824 FS6 302 | 12.5 | 5.6 | 0.9 |
| 1306-3 pUC18-824 FS6 303 | 11.0 | 5.4 | 1.3 |

Cellulose production in these recombinant strains was similar to that observed for the parent strains.

It was also observed that cellulose synthase activity was suppressed in strains carrying a disrupted cellulose synthase B gene. This gene was disrupted through the insertion of a 1.1 kb BamHI fragment encoding the streptomycin resistance gene at the internal BamHI site of the cellulose synthase B gene. The insertion of the streptomycin resistance gene interrupted the cellulose synthase B gene near its 5' end.

Example XVI

Chromosomal Promoter Replacement

Acetobacter appears to have a very efficient recombination system which may cause instability problems with any plasmid carrying a large segment of autonomous DNA. To overcome this potential problem, the operon can be overexpressed at the chromosomal level using heterologous control elements to drive transcription of the chromosomal cellulose synthase operon. The construction of plasmids pTac25-1, pLac21-7 containing heterologous promoters and the intermediate vectors used for their construction is described as follows and presented schematically in Figure 9.

A. Construction of MP11:Pcs:LFOI

Fifteen micrograms of pFS-1 DNA were digested with HindIII, releasing a 2.5 kb fragment carrying the cellulose synthase operon promoter. The digest was run on a 0.8% GTG agarose gel, the 2.5 kb fragment carrying the promoter was cut from the gel, electroeluted and ligated to double-stranded M13 MP phage DNA, which had previously been digested with HindIII. The ligation mixture was used to transform E. coli strain DG98. The transformed cells were plated on R17-3 plates with lawns of E. coli JM103 and incubated at 37°C overnight. Eleven of the resulting phage plaques were picked and inoculated in 3 ml of log phase JM103 diluted 1:100 with R2-6 medium (5.0 g Tryptome, 5.0 g yeast extract, 5.0 g NaCl and 1.0 L distilled $H_2O$, pH 6.9). These cultures were incubated at 37°C for six hr and spun down in Eppendorf tubes. The supernatant containing free phage was stored at 4°C while mini-screen DNA was prepared from the cell pellets by the alkaline lysis method. The mini-screen DNA was analyzed by restriction digestion with the enzymes HindIII and Bg1II. One clone was chosen and was designated MPII:Pcs. The supernatant from this clone was used to infect JM103. A 15 ml culture of the JM103 MPII:Pcs was grown for six hr, spun down, and single-stranded phage DNA was isolated from the supernatant.

The phage DNA MPII:Pcs was mutagenized with Oligonucleotide e LFOI (5'-GAATATATAACGGAGCTCCCG-GGATCCACCTGTTTTACC-3'), which contains the restriction site sequences for the enzymes SstI, SmaI, and BamHI flanked by 12 bp of cellulose synthase operon promoter sequence on each side. One picomole of the single-stranded phage DNA MP11:Pcs was incubated with 10 pmoles of LFOI at 68°C for 5 min. and then allowed to anneal with the promoter sequences for 30 min. at 37°C. The annealed molecules were then extended to form complete double-stranded DNAs by adding dNTPs to 0.5 mM and the Klenow fragment of DNA polymerase 1 to 0.25 units/ul. The extension proceeded at 4°C for 30 min., and then at 37°C for one hr.; it was then heated to 68°C for 10 min. and the mixture used to transform JM103 competent cells.

The transformation mixture was plated with JM103 lawn cells on R2-4 plates with 3 ml R-17 (10.0 g n-Z amine, type A, 5.0 g NaCl, 0.04 g X-gal in 2.0 ml DMF), 10 mM $MgCl_2$, 0.7% top agarose. Phage plaques were lifted from these plates onto nitrocellulose filters and the plates stored at 4°C. The filters were baked at 80°C for two hr. and hybridized with the $^{32}P$-labelled oligonucleotide probe LF02. LF02 contains a subset of the sequence of the oligonucleotide LF01 used for mutagenesis, the three restriction site sequences with only 2 bp of cellulose synthase of LF02 is 5'-GGGAGCTCCCGGGATCCAC-3'. Hybridization was carried out at 58°C. The filters were washed at 58°C with sequential 5 min washes of 5x SSC, 2x SSC, and 2x SSC containing 0.1% SDS. Kodak X-OMAT AR film was exposed to the filters for 60 hr. After 60 hr., dark spots appeared on the developed film which corresponded to the lifted plaques still present on the plates. Sixteen plaques which corresponded to dark spots on the film were isolated and the RF DNAs were analyzed by restriction digestion with the enzymes HindIII, BamHI, and SstI. MP11:Pcs:LFOI corresponded to the map provided in Figure 9. The introduced restriction sites in MP11:Pcs:LFOI are for substitution of heterologous promoters. The flanking regions serve as the sites for homologous recombination between the plasmid and the Acetobacter chromosome in gene replacement.

The phage supernatant from culture MP11:Pcs:LFOI was plaque purified, checked again for the appropriate restriction pattern, then used for infection and preparation of double-stranded cesium chloride gradient purified DNA.

B. Construction of pACYC184:Pcs

Twenty micrograms of MP11:Pcs:LFOI DNA were digested with 200 units of HindIII, and the 2.5 kb fragment containing the cellulose synthase promoter region was gel purified. This fragment was ligated to HindIII-digested pACyl84 (New England Biolabs). The ligation mixture was transformed into MM294 competent cells and plated on R2-4 plates containing Cm at 20 ug/ml. The plates were incubated at 37°C overnight and over 15,000 $Cm^R$ colonies appeared. To test for inactivation of the tetracycline resistance gene of pACYC184 by the insertion of the Pcs fragment, 66 of these colonies were patched on to R2-4 plates with ampicillin at 50 ug/ml and R2-4 plates with tetracycline at 15 ug/ml and incubated at 37°C overnight. Six of the sixty-six $Cm^R$ $Amp^R$ colonies showed sensitivity to tetracycline on the patch test. Mini-prep DNA was isolated from these six clones, digested with HindIII. Three of the plasmids showed one 2.5 kb HindIII insert while the other three showed two or more. The plasmid designated pACYC184:Pcs showed a single 2.5 kb HindIII insert.

Forty ug of pACYC184:Pcs DNA was partially digested with 4 units of BamHI and gel purified. The approximately 6.7 kb fragment, containing linearized plasmid molecules cut at one of the two BamHI sites, was isolated and then digested to completion with SstI. The fragments were gel purified and the 6.7 kb BamHI-SstI fragment, was cut from the gel, electroeluted into 0.1x TEA, extracted with phenol/chloroform, precipitated with sodium acetate in ethanol, and resuspended in Tris-EDTA buffer.

C. Introduction of a Unique SstI Site into pBR322

Ten micrograms of pBR322 DNA was digested to completion with the restriction endonuclease AlwNI, and the ends were made blunt. SstI (SacI) linker oligonucleotides from New england Biolabs were ligated with T4 ligase to the blunt-ended pBR322 cut fragment under standard conditions. The ligation mixture was digested directly with 5 units of SstI, gel purified and then ligated to itself with T4 DNA ligase under standard conditions. This ligation mixture was used to transform MM294 competent cells. One culture, MM294 pALF20, gave DNA which was linearized with SstI and was approximately 4.4 kb in length. This plasmid was then used to accommodate the lac and tac promoters.

D. Annealing of Oligonucleotides

Oligonucleotides were synthesized to form the tac and lac UV5 promoters. Each oligonucleotide contains the sequence for one strand of the promoter, with one strand of an EcoRI half site at one end and one strand of a HindIII half site at the other. The synthesized oligonucleotides were suspended in $H_2O$ to a concentration of 100 pmoles/ul. Two hundred pmoles of each oligonucletide were treated with 9 units of polynucleotide kinase in a 20 ul reaction containing 1 mM ATP at 37°C for 30 min. Once kinased, the oligonucleotides were mixed together in pairs then heated for 15 min. to 68°C to unpair any secondary structure. The oligonucleotides were then allowed to anneal together by cooling to 37°C and incubating for 30 min. After 30 min. the annealed oligonucleotides were included in ligation reactions with the pALF20 EcoRI/HindIII fragment isolated as described below.

E. Preparation of Vector

Twenty ug of the plasmid pALF20 were digested with 100 units of EcoRI and 100 units of HindIII at 37°C for 1.5 hr. The reaction was run on a 0.8% GTG agarose gel and the approximately 4.4 kb fragment, corresponding to linearized DNA, was cut from the gel. The fragment was electroeluted.

F. Ligations

Each pair of annealed oligonucleotides was ligated to the HindIII/EcoRI-digested pALF20 DNA in a reaction containing 0.1 mM ATP with an insert to vector ratio of 3:1 at 16°C for 3.5 hr. As a control, the vector EcoRI/HindIII-digested pALF20 was ligated to itself under the same conditions.

G. Transformations

Each ligation mixture was transformed into DG101 competent cells and plated on R2-4 plates containing tetracycline at 15 ug/ml. The transformation with the vector ligating on itself gave six tetracycline resistant colonies. pALF20:lacUV5 gave 30 tetracycline resistant colonies, pALF20:tac gave 99. Cesium chloride ethidium bromide gradient purified DNA was prepared from three clones from the pALF20:lacUV5 transformation, designated pLacl9, pLac20, and pLac21, and the two clones from the pALF20:tac transformation, designated pTac24 and pTac25. These plasmids were sequenced to determine the presence of an annealed oligonucleotide promoter insert:

pLac21: Contained the lacUV5 sequence with no errors.

pTac25: Contained the tac promoter sequence with a 1 bp mismatch from G to A at the -47 position of the promoter.

Plasmids pTac25 and pLac21 were selected to continue construction. Ten ug of each plasmid were digested to completion with SstI and then with BamHI. The approximately 1.5 kb fragments containing the ampicillin resistance gene attached to a heterologous promoter (tac or lac) were cut out of the gels, and electroeluted.

H. Ligations

The approximately 6.7 BamHI-partial SstI pACY-C184:Pcs vector fragment (isolated in Section B) was ligated to the BamHI/SstI ampicillin resistance heterologous promoter fragments from pLac21 and pTac25 in two separate ligation reactions with insert-to-vector ratios of 7:1 and an ATP concentration of 0.2mM at 16°C for 24 hr. These two ligation mixtures were used to transform DG101 competent cells. The transformations yielded 786 ampicillin resistant transformants for pACYC184:Pcs:Lac21 and -803 ampicillin resistance transformants from pACyl84:Pcs:Tac25. Cultures were grown in R2 with ampicillin at 50 ug/ml from 9 colonies from each transformation. Alkaline lysis mini-prep DNA was isolated from these cultures and analyzed by restriction digestion with the enzymes BamHI, HindIII, and SstI. DNAs which corresponded to the restriction map for the plasmids designated pLac21-7 and pTac25-1 in Figure 9 were identified. These plasmids were used to transform 1306-21 electrocompetent cell stock. The resulting strains were subjected to Southern blot analysis to verify the chromosomal configuration of these strains, and then used in fermentation experiments to increase levels of cellulose synthase activity and cellulose production.

These two strains, along with the untransformed host strain 1306-21, were tested using standard protocols. Strains were tested in duplicate for cell growth, cellulose production and in vitro cellulose activity for days 1, 2, and 5. One hundred twenty-five milliliter waffled flasks were inoculated with a 2% cellulose containing seed media. Standard flask media containing R70-2 with 10 uM $FeCl_3$, 25 mM DMG, 1% TYE and 3% glucose was used.

The cultures for the enzyme assays were harvested, the cells passed through cheese cloth, and the cells were washed using previously described protocols. The pellets were frozen as the samples were collected. The cells were sonicated and then assayed for cellulose synthase activity, production of cellulose, and cell growth.

The results of this experiment demonstrate that one can replace the Acetobacter chromosomal promoter of the cellulose synthase operon with an E. coli promoter and still obtain in vitro cellulose synthase activity and cellulose production. The in vitro cellulose synthase specific activity of the tac-promoter construct was similar to that of the wild-type cells throughout the fermentation, both in the activity of day-old cultures, and in the decline in activity in the two and five day-old cultures, while the activity of the lac-promoter construct was lower than that of the wild-type strain. Its activity also declined between the first and second days.

Cell growth and cellulose production of the tac-promoter construct were experimentally identical to that of the control. The lac-promoter construct was markedly lower in cellulose production, but not in cell growth, as compared to the control. This data suggests that production of cellulose in the lac-promoter strain was limited by the in vivo activity of cellulose synthase.

Thus, as demonstrated herein, promoters of varying strength may give rise to different enzyme activities and cellulose to cell ratios.

Example XVII

Experiments With Cellulose Synthase D Gene

A. Cloning of a 3.7 kb Fragment Containing Gene C and D Sequences into pACYC184

Twenty ug of pACYC184 DNA were digested with 200 units of EcoRV and 200 units of BamHI in a total volume of 300 ul at 37°C for 2 hr. The DNA was treated with 14 units of calf intestinal alkaline phosphatase at 37°C for 30 min., then run on a 1% GTG agarose gel. The approximately 4 kb EcoRV/BamHI fragment was cut from the gel, and electroeluted.

Fifty ug of pBR322:5.5 T19G9 DNA were digested to completion with SmaI, and then digested with BamHI. (The 5.5 kb BamHI fragment from TRT11-4 (Example XIV.C) was cloned into the BamHI site of pBR322 to construct pBR322: 5.5 T19G9.) The digested DNA was run on a 1% GTG agarose gel, the approximately 3.7 kb fragment (Figure 1 nucleotides 6341-10164), containing the 3' portion of gene C and the complete gene D, was cut from the gel and electroeluted into a small volume of 0.1X TEA.

Approximately 2 ug equivalents of the pACYC184 BamHI/EcoRV fragment were ligated to approximately 25 ug equivalents of the 3.7 kb BamHI/SmaI fragment, at an insert-to-vector ratio of approximately 3:1.

This ligation mixture was used to transform MM294 competent cells under standard conditions. The transformed cells were selected on R2-4 plates containing 20 ug/ml Cm and incubated at 37°C overnight. $Cm^R$ transformants were obtained and one or more clones were picked for preparation of alkaline lysis mini-screened DNA. Clone pACYC184: 3.7 was shown to carry a 3.7 kb SmaI-BamHI insert containing the 3' end of gene C and all of gene D, including the stem and loop terminator structure at the end of the operon.

B. Interruption of Gene D Sequence

Twenty ug of pACYC184:3.7 DNA were digested to completion with EcoRV extracted with phenol/chloroform, precipitated and then resuspended in 20 ul of Tris-EDTA. Five ug of pBR322 DNA was digested to completion with EcoRI and AlwNI. The DNA was precipitated and the sticky ends filled-in with Klenow in the presence of all four dNTPs.

The 5 ug equivalents of pBR322 $Amp^R$ fragment DNA were ligated to 1 ug equivalent of the pACYC184:3.7 EcoRV-digested DNA. The ligation mixture was used to transform MM294 competent cells under standard conditions. The transformation was plated on R2-4 Amp 50 plates. $Amp^R$ colonies were obtained and cultured. Cells were harvested from each culture and alkaline lysis mini-prep DNA was isolated from the cultures. One of the cultures, designated pDI-2, was chosen for transformation of 1306-21. pDI2 contained the Amp resistant fragment from pBR322 inserted in the EcoRV site of pACYC184:3.7, interrupting gene D. Cesium chloride ethidium bromide gradient pDI-2 DNA was prepared. 10 ug of this DNA were digested to completion with XbaI. Forty ul of electrocompetent 1306-21 cells were added to the digested DNA and the cells electroporated under standard conditions. One ml of R20-2 was added to the electroporated cells and the mixture plated immediately on R20-2 Amp 100 Cm 20 plates.

After four days incubation at 30°C, approximately 100,000 $Amp^R$ colonies appeared on the plates. These transformants appeared as weak cellulose producers. Similar results were obtained for the transformants wherein the entire cellulose synthase D gene was deleted. These findings suggest that the cellulose synthesis D gene does play a role in cellulose synthesis.

Example XVIII

Purification of Cellulose Synthase

Strain 1306-27 was grown on a 400 ml 4% fructose, 1% yeast extract, 0.5% Bactopeptone, 0.3% $NaH_2PO_4$ medium, at pH 5.0 in 1000 ml baffled flasks, for 24 hr. Growth media was removed from the cells by washing twice with buffer (50 mM $K_2HPO_4$, pH 6.0). About 14 g of dry cells were prepared.

Cell Membranes

Cells (25 mg cells/mL) were ruptured in a French press in the presence of polyethyleneglycol (PEG) and TME buffer, 20% PEG (w/v). The broken cells were centrifuged (12,000 x g, for 10 min.) and the pellet was resuspended in TME buffer to the same volume. This suspension was homogenized (Potter Elevehjem homogenizer) and the suspension was centrifuged (12,000 x g, for 10 min.). The resulting pellet contained the cellulose synthase activity. This sample (P-PEG) was resuspended in TME buffer to a concentration corresponding to 50 mg cell weight per ml and was frozen in liquid nitrogen and stored at - 80°C.

Trypsin Treatment

P-PEG was centrifuged (12,000 x g, 10 min.) and the pellet was suspended in 0.1 M Tris, pH 8.3, 20% sucrose to 10 mg cells dry weight/ml. A 1% volume of 8 mg/ml trypsin (Sigma) was added and the preparation incubated with gentle shaking for 1 hr. at 4°C. A 1% volume of 2 mg/ml trypsin inhibitor was added and the preparation was incubated for 15 min. on ice. After centrifugation (100,000 x g, for 30 min.) the pellet (TT-P-PEG) was stored frozen at -80°C.

Solubilization

A 10% solution of digitonin (Serva, Westbury, NY) in TME buffer was prepared by heating for 5-10 min. in a boiling bath and the preparation was then cooled to 4°C. The TT-P-PEG pellet was suspended to 1/10 the original volume in TME buffer containing 2% digitonin. The suspension was sonified at 4°C for 2 min. using a MSE Model 140 sonifier. Sonification was carried out on 30 ml portions in 30 sec. sonic pulses with intermittent cooling. The suspension was shaken and mixed gently for 90 min. and then centrifuged at 200,000 x g for 60 min. The supernatant contained approximately 50% of original cellulose synthase activity. When frozen and stored as above, activity was retained for several months.

Enzyme Concentration

The solubilized enzyme was concentrated 5-7 fold using Amicon cones (Filter 100K) to an activity of 10-16 enzyme units/ml (unit = 1 nanomole/min). The concentrated enzyme was kept overnight at 4°C or frozen as above.

Enzyme-product Entrapment

To the bottom of each of six 40 ml centrifuge tubes for the Contron TST-28 (swinging bucket) rotor, were added 26 ml of the glycerol-containing cushion (TME buffer (pH 8.5) containing 12-13% glycerol, 1 mM UDPG and 15 umole c-di-GMP) and 10 ml reaction mixture (50 ml solubilized enzyme, 6.25 mmole Tris-HC buffer, pH 9.6, 340 umole $CaCl_2$, 1 mmole $MgCl_2$, 0.6 umole c-di-GMP, and 60 umole UDPG to a final volume of 61 ml) were gently layered on it. The tubes were incubated for 15 min. at 30°C, then placed on ice for 2.5 hrs., and finally centrifuged in a TST-28 Contron rotor for 30 min. at 20,000 rpm. (The centrifuge was set to decelerate to 350 rpm in 3-4 min.) The supernatants were carefully decanted. The pellets were combined and suspended in 15 ml TME with a manual homogenizer and recentrifuged. The final pellet was suspended in 5 ml TME. Before SDS-PAGE, the entrapped enzyme was washed once by centrifugation. The entrapped enzyme was stable for several weeks when frozen and stored as above. The activity yield of entrapped enzyme was about 45%.

Separation of Proteins on SDS-PAGE

Entrapped enzyme, containing 12-15% of original cellulose synthase activity, was dissolved in Laemmli's sample buffer (containing DTT), and subjected to SDS-PAGE in 10% acrylamide slab gels. The peptide bands were visualized by Coomassie-Blue staining and excised. Four major bands were observed, Band A 90-95 kd, Band B 65-68 kd, Band C 58-60 kd, and Band D 54-56 kd.

Recovery of the Protein from the SDS-PAGE Gel Slices

The proteins were separated from the SDS-PAGE gel slices by electroelution, using a method modified from Hunkapiller, et al. (Methods in Enzymology, 1983, 91:227-247). The method was adapted to result in a lower final SDS concentration. The modifications were: 1) substitution of elution buffer (0.1% SDS in 0.05 M $NH_4HCO_3$) for the soaking buffer (2% SDS in 0.05 M $NH_4HCO_3$); and 2) when replacing the elution buffer (0.1% SDS in 0.05 M $NH_4HCO_3$) in the apparatus with dialysis buffer (0.02% SDS in 0.01 M $NH_4HCO_3$), most of the buffer in the sample cell was also replaced, carefully avoiding disturbing the sample.

Example XIX

N-Terminal Amino Acid Sequence of The Polypeptides Protein Isolated From The Cellulose Synthase Preparation

The first 18 amino acids of the 90-95 kd protein and the first 16 amino acids of the 65-68 kd protein purified from *Acetobacter* strain 1306-27 were sequenced by automated Edman degradation on an Applied Biosystems model 470A Protein Sequencer, using the reagents and protocol supplied by the manufacturer. The 18 amino acids of the 90-95 kd protein match an amino acid sequence predicted from the DNA sequence of cellulose synthase obtained as described above. The match begins with the alanine residue indicated in Figure 1. The amino-terminus obtained after purification may not be the actual *in vivo* N-terminus, but may reflect proteolysis at the lysine preceding this alanine. The deduced sequence of the cloned gene codes for a protein of 83 kd. Some additional peaks for the early amino acids near the N-terminus were present, possibly due to contamination.

The quality of the sequence obtained for the 65-68 kd protein was not as high as that obtained for the 90-95 kd protein, but a good match may be made between the 65-68 kd protein N-terminal sequence and the amino acids (designated by the brackets) from the sequence predicted in Figure 1. Thus, the 65-68 kd protein appears to be a proteolytic fragment of the 90-95 kd protein.

The following cultures have been deposited at the American Type Culture Collection, (ATCC) Rockville, MD, USA, under the terms of the Budapest Treaty on the International Recognition of the Deposit of Microorganisms for the Purposes of Patent Procedure and Regulations thereunder (Budapest Treaty) and are thus maintained and made available according to the terms of The Budapest Treaty. Availability of such strains is not to be construed as a license to practice the invention in contravention of the rights granted under the authority of any government in accordance with its patent laws.

The deposited cultures have been assigned the indicated ATCC deposit numbers. The cultures have also been deposited with the Master Culture Collection (CMCC) of Cetus Corporation, Emeryville, CA, USA, the assignee of the present application, and assigned the indicated CMCC deposit numbers:

| Culture | CMCC No. | ATCC No. |
|---|---|---|
| Acetobacter 1306-24 pUC18-824 FS6 | 3538 | 67925 |
| E. coli DG101 pUC18-824 FS6 | 3581 | 67926 |
| Acetobacter strain 1306-3 | 1909 | 53264 |
| Acetobacter strain 1306-11 | 2145 | 53263 |
| Acetobacter strain 1306-21 | 2618 | 53524 |
| E. coli DG98 | 1965 | 39768 |
| E. coli DG101 pUC18-824 pABCD | | 68264 |

While the present invention has been illustrated above by certain specific embodiments, it is not intended that these specific examples limit the scope of the invention as described in the appended claims.

## Claims

1. An isolated native, cloned recombinant or synthetic polynucleotide sequence encoding a bacterial cellulose synthase operon, wherein with reference to Figure 1 said polynucleotide comprises four contiguous genes, the first gene corresponding substantially to nucleotides 328 to 2589, the second gene corresponding substantially to 2594 to 4999, the third gene corresponding to substantially nucleotides 5005 to 8961, and the fourth gene corresponding substantially to nucleotides 8964 to 9431.

2. A polynucleotide of claim 1 which is derived from the genome of Acetobacter.

3. A polynucleotide of claim 1 or claim 2 which further comprises a promoter located adjacent to and upstream from said operon.

4. An isolated native, cloned recombinant or synthetic polynucleotide substantially corresponding to the nucleotide sequence for cellulose synthase A, cellulose synthase B, cellulose synthase C, or cellulose synthase D, in each case as shown in Figure 1.

5. A polynucleotide of claim 4 which is operatively linked to a control sequence for expression.

6. A polynucleotide of claim 5 which further comprises a promoter located adjacent to and upstream from said polynucleotide corresponding to the nucleotide sequence for cellulose synthase A, B, C or D.

7. Recombinant host cells transformed with the polynucleotide of any preceeding claim.

8. A method for producing bacterial cellulose synthase comprising culturing transformed cells of claim 7 under conditions suitable for the expression of bacterial cellulose synthase, and recovering the expressed bacterial cellulose synthase from the culture.

9. An isolated, recombinant protein encoded by a polynucleotide as defined in claim 4 any or any one of claims 1 to 6.

10. A method for increasing cellulose production in a recombinant microorganism, which method comprises:

(a) transforming a suitable microorganism with a vector comprising at least one gene substantially corresponding to the nucleotide sequence for cellulose synthase A, cellulose synthase B, cellulose synthase C, or cellulose synthase D, in each case as shown in Figure 1, and
(b) culturing said transformed microorganism under conditions suitable for production of cellulose.

**11.** A recombinant DNA vector comprising:

(a) at least one gene derived from the cellulose synthase operon, said gene comprising a sequence substantially corresponding to the nucleotide sequence for cellulose synthase A, cellulose synthase B, cellulose synthase C, or cellulose synthase D, in each case as shown in Figure 1.

(b) A functional Acetobacter origin of replication-containing fragment of that 3.5 kb plasmid DNA which is obtainable by SacI digestion of ATCC 67925 or ATCC 67926, and

(c) one or more DNA segments that convey resistance to at least one antibiotic when transformed into a sensitive host cell that is susceptible to transformation, cell division and culture.

**12.** A recombinant DNA vector of claim 11 which is either that vector obtainable from ATCC 67925 or ATCC 67926, or is identical to said vector except that the cloned 8.3 kb SmaI restriction fragment is in the reverse orientation.

**Patentansprüche**

**1.** Isolierte, native, clonierte rekombinante oder synthetische Polynucleotidsequenz, die ein bakterielles Cellulosesynthase-Operon codiert, wobei unter Bezugnahme auf Figur 1 das Polynucleotid vier benachbarte Gene umfaßt, wobei das erste Gen im wesentlichen den Nucleotiden 328 bis 2589, das zweite Gen im wesentlichen den Nucleotiden 2594 bis 4999, das dritte Gen im wesentlichen den Nucleotiden 5005 bis 8961 und das vierte Gen im wesentlichen den Nucleotiden 8964 bis 9431 entspricht.

**2.** Polynucleotid nach Anspruch 1, das aus dem Genom von Acetobacter stammt.

**3.** Polynucleotid nach Anspruch 1 oder Anspruch 2, das weiter einen zu dem Operon benachbarten und davon stromaufwärts liegenden Promotor umfaßt.

**4.** Isoliertes, natives, cloniertes rekombinantes oder synthetisches Polynucleotid, das im wesentlichen der Nucleotidsequenz für die Cellulosesynthase A, Cellulosesynthase B, Cellulosesynthase C oder Cellulosesynthase D entspricht, in jedem Fall wie in Figur 1 gezeigt.

**5.** Polynucleotid nach Anspruch 4, das funktionell mit einer Kontrollsequenz für die Expression verbunden ist.

**6.** Polynucleotid nach Anspruch 5, das weiter einen Promotor umfaßt, der benachbart zu und stromaufwärts des Polynucleotids liegt, das der Nucleotidsequenz für die Cellulosesynthase A, B, C oder D entspricht.

**7.** Rekombinante Wirtszellen, die mit dem Polynucleotid nach einem der vorhergehenden Ansprüche transformiert sind.

**8.** Verfahren zur Herstellung einer bakteriellen Cellulosesynthase, umfassend die Züchtung der transformierten Zellen nach Anspruch 7 unter Bedingungen, die für die Expression der bakteriellen Cellulosesynthase geeignet sind, und Gewinnung der exprimierten bakteriellen Cellulosesynthase aus der Kultur.

**9.** Isoliertes rekombinantes Protein, das von einem Polynucleotid gemäß der Definition in Anspruch 4 oder einem der Ansprüche 1 bis 6 codiert wird.

**10.** Verfahren zur Erhöhung der Celluloseproduktion in einem rekombinanten Mikroorganismus, umfassend:

(a) Transformation eines geeigneten Mikroorganismus mit einem Vektor, der mindestens ein Gen umfaßt, das im wesentlichen der Nucleotidsequenz für Cellulosesynthase A, Cellulosesynthase B, Cellulosesynchase C oder Cellulosesynthase D entspricht, in jedem Fall wie in Figur 1 gezeigt, und

(b) Züchtung des transformierten Mikroorganismus unter für die Produktion von Cellulose günstigen Bedingungen.

11. Rekombinanter DNA-Vektor, umfassend:

(a) mindestens ein Gen, das von dem Cellulosesynthase-Operon stammt und eine Sequenz umfaßt, die im wesentlichen der Nucleotidsequenz für Cellulosesynthase A, Cellulosesynthase B, Cellulosesynthase C oder Cellulosesynthase D entspricht, in jedem Fall wie in Figur 1 gezeigt;
(b) ein funktionelles, den Acetobacter-Replikationsursprung enthaltendes Fragment der Plasmid-DNA von 3,5 kb Länge, das durch eine SacI-Spaltung aus ATCC 67925 oder ATCC 67926 erhältlich ist, und
(c) eine oder mehrere DNA-Segmente, die gegenüber mindestens einem Antibiotikum Resistenz verleihen, wenn sie in eine sensitive, einer Transformation, Zellteilung und Züchtung zugängliche Wirtszelle transformiert werden.

12. Rekombinanter DNA-Vektor nach Anspruch 11, der entweder der Vektor ist, der aus ATCC 67925 oder ATCC 67926 erhältlich ist, oder der mit diesem Vektor identisch ist, ausgenommen daß das clonierte SmaI-Restriktionsfragment von 8,3 kb Länge in der umgekehrten Orientierung vorliegt.

## Revendications

1. Séquence polynucléotidique isolée native, recombinante par clonage ou synthétique, codant pour un opéron cellulose-synthase bactérien, ledit polynucléotide comprenant, en référence à la figure 1, quatre gènes contigus, le premier gène correspondant essentiellement aux nucléotides 328 à 2589, le second gène correspondant essentiellement à 2594 à 4999, le troisième gène correspondant essentiellement aux nuclétotides 5005 à 8961 et le quatrième gène correspondant essentiellement aux nucléotides 8964 à 9431.

2. Polynucléotide selon la revendication 1 qui est dérivé du génome d'Acetobacter.

3. Polynucléotide selon la revendication 1 ou la revendication 2 qui comprend en outre un promoteur adjacent audit opéron et localisé en amont de celui-ci.

4. Polynucléotide isolé natif. recombinant par clonage ou synthétique correspondant essentiellement à la séquence nucléotidique de la cellulose synthase A, de la cellulose synthase B, de la cellulose synthase C, ou de la cellulose synthase D, chaque cas étant tel que représenté figure 1.

5. Polynucléotide selon la revendication 4 qui est lié de manière opérante à une séquence de contrôle de l'expression.

6. Polynucléotide selon la revendication 5 qui comprend en outre un promoteur adjacent audit polynucléotide correspondant à la séquence nucléotidique de la cellulose synthase A, B, C ou D, et localisé en amont de celui-ci.

7. Cellules hôtes recombinantes transformées par le polynucléotide selon l'une quelconque des revendications précédentes.

8. Procédé de production de cellulose synthase bactérienne comprenant la culture des cellules transformées selon la revendication 7 dans des conditions appropriées pour l'expression de la cellulose synthase bactérienne et par une récolte de la cellulose synthase bactérienne exprimée, de la culture.

9. Protéine isolée, recombinante codée par un polynucléotide tel que défini dans la revendication 4 ou dans l'une quelconque des revendications 1 à 6.

10. Procédé pour augmenter la production de cellulose dans un microorganisme recombinant, ledit procédé comprenant :

a) la transformation d'un microorganisme adapté avec un vecteur comprenant au moins un gène correspondant essentiellement à la séquence nucléotidique de la cellulose synthase A, de la cellulose synthase B, de la cellulose synthase C, ou de la cellulose synthase D, chaque cas étant tel que représenté figure 1 ; et
b) la culture dudit microorganisme transformé dans des conditions appropriées à la production de cellulose.

**11.** Vecteur d'ADN recombinant comprenant :

(a) au moins un gène dérivé de l'opéron cellulose synthase, ledit gène comprenant une séquence correspondant essentiellement à la séquence nucléotidique de la cellulose synthase A, de la cellulose synthase B, de la cellulose synthase C, ou de la cellulose synthase D, chaque cas étant tel que représenté figure 1,
(b) un fragment contenant une origine de réplication fonctionnelle d'Acetobacter de l'ADN plasmidique de 3,5 kb qui peut être obtenu par digestion par SacI de ATCC 67925 ou ATCC 67926, et
(c) un ou plusieurs segments d'ADN qui confère(nt) une résistance à au moins un antibiotique lorsqu'il est transformé dans une cellule hôte sensible qui est susceptible de transformation, de division cellulaire et de culture.

**12.** Vecteur d'ADN recombinant selon la revendication 11, qui est soit un vecteur pouvant être obtenu à partir des souches ATCC 67925 ou ATCC 67926, ou qui est identique audit vecteur à l'exception du fait que le fragment de restriction SmaI de 8,3 kb cloné est orienté en sens inverse.

FIG. 1 - 1

```
   1 GGCTGGCCGCCCCGTGCCGACCGACAACTCCCCGACCCTGACCGAAGTGTTCATGACCCTTGGTGGTCGGGCCACGGACCGGTTGGTGCC 90

  91 CAAGCCCAGCCTGCGCGATGCCCTGTTGCGCAAGCGTGAAGACGCGAACGGCGACTCCTGAAACCGTGCCGGGGGCGACCTGCTCCCGGC 180
                                                            ↓
 181 ATGTCAGAGGAAAGAAGGGGGAAGGTTTTCCCCGCCCCGCATCGCTGCGGGCCGAAAGGCGACATGACGGACCGAATGCGTCTGACGGTT 270
                                                                          |——→ Gene A
 271 TTCTTTTGAATATATAACGACCTGTTTTACCAGTATTTATTATCGGACGAGCTATTGATGTCAGAGGTTCAGTCGCCAGTACCCGCGGAG 360
                                                                         MetSerGluValGlnSerProValProAlaGlu

 361 AGTAGGCTAGACCGCTTTTCCAACAAGATACTGTCACTGCGTGGGGCCAACTATATAGTTGGAGCGCTGGGGCTTTGTGCACTTATCGCC 450
     SerArgLeuAspArgPheSerAsnLysIleLeuSerLeuArgGlyAlaAsnTyrIleValGlyAlaLeuGlyLeuCysAlaLeuIleAla

 451 GCAACCACGGTCACGCTGTCCATTAATGAGCAGCTGATTGTGGCACTTGTGTGTGTGCTCGTCTTTTTTATTGTCGGGCGCGGCAAGAGC 540
     AlaThrThrValThrLeuSerIleAsnGluGlnLeuIleValAlaLeuValCysValLeuValPhePheIleValGlyArgGlyLysSer

 541 CGCCGCACCCAGATCTTTCTCGAGGTGCTCTCGGCGCTGGTCTCCCTGCGTTACCTGACATGGCGCCTGACCGAAACGTTGGACTTCGAT 630
     ArgArgThrGlnIlePheLeuGluValLeuSerAlaLeuValSerLeuArgTyrLeuThrTrpArgLeuThrGluThrLeuAspPheAsp

 631 ACATGGATTCAGGGCGGGCTGGGCGTGACCCTGCTCATGGCCGAACTCTATGCCCTGTACATGCTGTTTCTCAGCTATTTCCAGACAATC 720
     ThrTrpIleGlnGlyGlyLeuGlyValThrLeuLeuMetAlaGluLeuTyrAlaLeuTyrMetLeuPheLeuSerTyrPheGlnThrIle

 721 CAGCCACTTCATCGCGCGCCGCTGCCCCTGCCGGACAATGTTGATGACTGGCCAACCGTCGACATCTTCATCCCGACCTATGATGAACAG 810
     GlnProLeuHisArgAlaProLeuProLeuProAspAsnValAspAspTrpProThrValAspIlePheIleProThrTyrAspGluGln

 811 CTCAGCATCGTGCGCCTGACCGTGCTGGGCGCGCTGGGCATCGACTGGCCGCCCGATAAAGTGAATGTCTATATCCTTGATGATGGTGTG 900
     LeuSerIleValArgLeuThrValLeuGlyAlaLeuGlyIleAspTrpProProAspLysValAsnValTyrIleLeuAspAspGlyVal

 901 CGCCCCGAATTTGAACAGTTTGCCAAGGATTGCGGCGCTCTCTACATCGGGCGCGTCGACAGTTCACACGCCAAGGCGGGTAACCTCAAC 990
     ArgProGluPheGluGlnPheAlaLysAspCysGlyAlaLeuTyrIleGlyArgValAspSerSerHisAlaLysAlaGlyAsnLeuAsn

 991 CACGCCATTAAGCGGACAAGCGGCGATTACATCCTCATCCTGGATTGTGACCATATTCCGACACGCGCGTTCCTGCAGATCGCGATGGGC 1080
     HisAlaIleLysArgThrSerGlyAspTyrIleLeuIleLeuAspCysAspHisIleProThrArgAlaPheLeuGlnIleAlaMetGly

1081 TGGATGGTCGCAGACCGCAAGATCGCCCTGATGCAGACGCCGCATCACTTCTACTCCCCCGATCCGTTCCAGCGTAACTTGGCCGTGGGG 1170
     TrpMetValAlaAspArgLysIleAlaLeuMetGlnThrProHisHisPheTyrSerProAspProPheGlnArgAsnLeuAlaValGly
```

FIG. 1-2

1171 TATCGCACCCCGCCGGAAGGCAACCTGTTCTACGGCGTCATTCAGGATGGTAACGACTTCTGGGATGCCACCTTCTTCTGCGGCTCGTGC 1260

TyrArgThrProProGluGlyAsnLeuPheTyrGlyValIleGlnAspGlyAsnAspPheTrpAspAlaThrPhePheCysGlySerCys

1261 GCCATCCTGCGGCGTGAAGCCATTGAATCGATCGGCGGCTTCGCGGTTGAAACCGTGACGGAAGATGCCCATACCGCCCTGCGCATGCAG 1350

AlaIleLeuArgArgGluAlaIleGluSerIleGlyGlyPheAlaValGluThrValThrGluAspAlaHisThrAlaLeuArgMetGln

1351 CGCCGTGGCTGGTCCACCGCCTACCTGCGCATTCCCGTTGCCAGTGGACTGGCCACCGAGCGACTGACAACCCATATCGGCCAGCGCATG 1440

ArgArgGlyTrpSerThrAlaTyrLeuArgIleProValAlaSerGlyLeuAlaThrGluArgLeuThrThrHisIleGlyGlnArgMet

1441 CGCTGGGCACGCGGCATGATCCAGATCTTCCGCGTGGACAACCCGATGCTCGGGGGCGGCCTGAAGCTTGGGCAGCGGCTGTGCTATCTC 1530

ArgTrpAlaArgGlyMetIleGlnIlePheArgValAspAsnProMetLeuGlyGlyGlyLeuLysLeuGlyGlnArgLeuCysTyrLeu

1531 TCGGCCATGACGTCGTTCTTCTTCGCCATTCCGCGCGTCATCTTCCTTGCCTCGCCGCTGGCGTTCCTGTTTTTCGGCCAGAACATCATC 1620

SerAlaMetThrSerPhePhePheAlaIleProArgValIlePheLeuAlaSerProLeuAlaPheLeuPhePheGlyGlnAsnIleIle

1621 GCCGCCTCGCCGCTGGCCGTGCTGGCCTACGCCATTCCGCACATGTTCCACTCCATCGCGACCGCCGCCAAGGTGAACAAGGGCTGGCGC 1710

AlaAlaSerProLeuAlaValLeuAlaTyrAlaIleProHisMetPheHisSerIleAlaThrAlaAlaLysValAsnLysGlyTrpArg

1711 TATTCGTTCTGGAGTGAAGTGTACGAAACCACCATGGCGCTGTTCCTGGTGCGCGTAACCATCATCACCCTGATGTTCCCCTCCAAGGGC 1800

TyrSerPheTrpSerGluValTyrGluThrThrMetAlaLeuPheLeuValArgValThrIleIleThrLeuMetPheProSerLysGly

1801 AAGTTCAACGTGACGGAAAAGGGTGGCGTGCTGGAGGAGGAAGAGTTCGACCTTGGCGCGACCTACCCCAACATCATTTTTGCCGGCATC 1890

LysPheAsnValThrGluLysGlyGlyValLeuGluGluGluGluPheAspLeuGlyAlaThrTyrProAsnIleIlePheAlaGlyIle

1891 ATGACGTTGGGGCTGCTGATCGGTCTGTTCGAACTGACCTTCCACTTCAACCAGCTCGCGGGCATTGCCAAGCGTGCTTACCTGCTGAAC 1980

MetThrLeuGlyLeuLeuIleGlyLeuPheGluLeuThrPheHisPheAsnGlnLeuAlaGlyIleAlaLysArgAlaTyrLeuLeuAsn

1981 TGCATCTGGGCGATGATCAGTCTCATCATCCTCCTTGCCGCCATTGCCGTGGGGCGTGAGACCAAGCAGGTCCGTTACAACCATCGTGTC 2070

CysIleTrpAlaMetIleSerLeuIleIleLeuLeuAlaAlaIleAlaValGlyArgGluThrLysGlnValArgTyrAsnHisArgVal

2071 GAGGCGCATATCCCGGTAACGGTTTATGAAGCACCGGTCGCGGGGCAGCCCAATACCTACCATAATGCGACACCGGGCATGACCCAGGAT 2160

GluAlaHisIleProValThrValTyrGluAlaProValAlaGlyGlnProAsnThrTyrHisAsnAlaThrProGlyMetThrGlnAsp

FIG. 1-3

```
                                        MK170
2161 GTCTCCATGGGTGGCGTTGCCGTCCACATGCCCTGGCCAGATGTCAGCACAGGACCAGTCAAGACACGCATTCATGCCGTGCTCGATGGC 2250
     ValSerMetGlyGlyValAlaValHisMetProTrpProAspValSerThrGlyProValLysThrArgIleHisAlaValLeuAspGly

2251 GAGGAGATCGATATTCCCGCCACCATGCTGCGCTGCAAGAATGGCAAGGCCGTGTTCACATGGACAATAATGACCTTGATACGGAACGC 2340
     GluGluIleAspIleProAlaThrMetLeuArgCysLysAsnGlyLysAlaValPheThrTrpAspAsnAsnAspLeuAspThrGluArg

2341 GATATTGTCCGCTTCGTGTTCGGGCGGGCCGATGCCTGGCTGCAATGGAATAATTATGAGGATGACAGACCGCTACGCAGTCTGTGGAGC 2430
     AspIleValArgPheValPheGlyArgAlaAspAlaTrpLeuGlnTrpAsnAsnTyrGluAspAspArgProLeuArgSerLeuTrpSer

2431 CTGCTGCTCAGCATTAAGGCGCTGTTCCGCAAAAAAGGCAAAATGATGGCCAATAGTCGTCCAAAAAGAAAACCACTTGCCCTACCGGTT 2520
     LeuLeuLeuSerIleLysAlaLeuPheArgLysLysGlyLysMetMetAlaAsnSerArgProLysArgLysProLeuAlaLeuProVal
                                                                             Gene A ->|  |-> Gene B
2521 GAGCGCAGGGAGCCCACAACCATCCAGAGTGGACAGACACAGGAAGGAAAGATCAGCCGTGCGGCCTCGTGATATGAAAATGGTGTCCCT 2610
     GluArgArgGluProThrThrIleGlnSerGlyGlnThrGlnGluGlyLysIleSerArgAlaAlaSer      MetLysMetValSerLe

2611 GATCGCGCTGCTGGTCTTTGCAACGGGCGCACAGGCTGCGCCTGTTGCCTCCAAGGCACCAGCCCCGCAGCCCGCAGGCTCAGACCTGCC 2700
     uIleAlaLeuLeuValPheAlaThrGlyAlaGlnAlaAlaProValAlaSerLysAlaProAlaProGlnProAlaGlySerAspLeuPr
                                                            ↑  ↑
2701 GCCCCTGCCTGCCGCGGCATCGCAGGCTGCCACGCCCGCTGCGGCAAGCGCGGACCAGCCCGCCACAACCGCCCCGGCGGCGGATGCCGC 2790
     oProLeuProAlaAlaAlaSerGlnAlaAlaThrProAlaAlaAlaSerAlaAspGlnProAlaThrThrAlaProAlaAlaAspAlaAl

2791 ATCAGCCAGTGCGGCTGATGCGGTCGTGGATAATGCCGAGAACGCCATTGCCGCGTCTGACGTGGCAACGGTGCATACATACTCCCTCAA 2880
     aSerAlaSerAlaAlaAspAlaValValAspAsnAlaGluAsnAlaIleAlaAlaSerAspValAlaThrValHisThrTyrSerLeuLy

2881 GGAGCTCGGTGCGCAGAGTGCCCTGAAAATGCAGGGCGCCGCCACGCTGCAGGGCCTGCAGTTCGGCATTCCGGCCGACCAGCTGGTCAC 2970
     sGluLeuGlyAlaGlnSerAlaLeuLysMetGlnGlyAlaAlaThrLeuGlnGlyLeuGlnPheGlyIleProAlaAspGlnLeuValTh

2971 GTCGGCACGGCTTATCGTGTCTGGAGCGATGTCGCCCAGCCTCCAGCCTGATACCAGCGCGGTCACGATCACGCTGAACGAGCAGTTCAT 3060
     rSerAlaArgLeuIleValSerGlyAlaMetSerProSerLeuGlnProAspThrSerAlaValThrIleThrLeuAsnGluGlnPheIl

3061 CGGCACGCTACGCCCGGACCCCACCCATCCTACATTTGGGCCGCTCTCGTTTGATATCAACCCCATCTTCTTCATCACGGGCAACCGGCT 3150
     eGlyThrLeuArgProAspProThrHisProThrPheGlyProLeuSerPheAspIleAsnProIlePhePheIleThrGlyAsnArgLe
```

FIG. 1 -4

```
3151 GAACTTCAGCTTCGCTTCAAGCTCGAAGGGCTGCACGGACCCCAGCAACGGATTGCTCTGGGCCAGCGTGTCCGAACATTCCGAACTGCA 3240
     uAsnPheSerPheAlaSerSerSerLysGlyCysThrAspProSerAsnGlyLeuLeuTrpAlaSerValSerGluHisSerGluLeuGl

3241 GATCACCACCATACCGCTTCCCCCGCGTCGTCAGCTCTCGCGCCTGCCCCAGCCGTTCTTCGACAAGAACGTAAAGCAGAAGATCGTCAT 3330
     nIleThrThrIleProLeuProProArgArgGlnLeuSerArgLeuProGlnProPhePheAspLysAsnValLysGlnLysIleValIl

3331 TCCGTTCGTTCTTGCACAGACATTTGATCCCGAAGTGCTGAAGGCGACCGGCATCCTGGCATCGTGGTTCGGCCAGCAGACCGATTTCCG 3420
     eProPheValLeuAlaGlnThrPheAspProGluValLeuLysAlaThrGlyIleLeuAlaSerTrpPheGlyGlnGlnThrAspPheAr

3421 TGGCGTTACCTTCCCGGTCTTCTCCACCATTCCGCAAACGGGCAATGCCGTTGTCGTTGGCGTGGCTGATGAACTGCCTTCCGCCCTCGG 3510
     gGlyValThrPheProValPheSerThrIleProGlnThrGlyAsnAlaValValValGlyValAlaAspGluLeuProSerAlaLeuGl

3511 GCGCCAGGCGGTCAATGGCCCCACGCTTATGGAAGTGGCCAATCCATCCGACCCCAACGGCACGGTGCTGCTCGTAACGGGGCGTGACCG 3600
     yArgGlnAlaValAsnGlyProThrLeuMetGluValAlaAsnProSerAspProAsnGlyThrValLeuLeuValThrGlyArgAspAr

3601 TGATGAAGTCATCACCGCGAGCAAGGGCATCGGCTTTGGCTCGAGCGCCCTGCCAACAGCCAACCGCATGGACGTGGCGCCGATTGATGT 3690
     gAspGluValIleThrAlaSerLysGlyIleGlyPheGlySerSerAlaLeuProThrAlaAsnArgMetAspValAlaProIleAspVa

3691 GGGCGCGCGTGTGGCCTATGACGCGCCCTCCTTCATTCCCACCAACCGTCCGGTCCGCCTTGGCGAACTGGTGCCAGACAGCGCCCTGCA 3780
     lGlyAlaArgValAlaTyrAspAlaProSerPheIleProThrAsnArgProValArgLeuGlyGluLeuValProAspSerAlaLeuGl

3781 GGCCCAGGGATACGCGCCGGGCGCACTCTCGGTGCCGTTCCGTGTCTCGCCCGATCTGTATACCTGGCGTGATCGACCGTACAAGCTGAA 3870
     nAlaGlnGlyTyrAlaProGlyAlaLeuSerValProPheArgValSerProAspLeuTyrThrTrpArgAspArgProTyrLysLeuAs

3871 CGTCCGTTTCCGCGCACCGCCAGGACCGATTGTCGATGTGTCGCGCTCGTCTCTCAACGTCGGTATCAACGATACCTATCTTGAGGCCTA 3960
     nValArgPheArgAlaProProGlyProIleValAspValSerArgSerSerLeuAsnValGlyIleAsnAspThrTyrLeuGluAlaTy

3961 TCCGCTGCGTGAGCCGGATTCAACGCTGGACCAGATCCTGCGGCGCGTGGGCCTGGGCCGTGGCGATGACAGCGTGCAGAAGCACACCAT 4050
     rProLeuArgGluProAspSerThrLeuAspGlnIleLeuArgArgValGlyLeuGlyArgGlyAspAspSerValGlnLysHisThrMe

4051 GCCCATCCCGCCCTACCGGGTTTTTGGCCAGAACCAGCTTCTGTTCTATTTCGAGATGGCGGCGATGGCCGAGCCGGGCTGCAAACCTGG 4140
     tProIleProProTyrArgValPheGlyGlnAsnGlnLeuLeuPheTyrPheGluMetAlaAlaMetAlaGluProGlyCysLysProGl

4141 CCCGAGCACGTTCCATATGAGTGTTGATCCGGATTCGACGATCGACCTGTCCAACTCCTATCATATCACGCGCATGCCCAACCTCGCCTT 4230
     yProSerThrPheHisMetSerValAspProAspSerThrIleAspLeuSerAsnSerTyrHisIleThrArgMetProAsnLeuAlaPh
```

FIG. I-5

```
4231 CATGGCCAGTGCGGGCTATCCGTTCACGACCTATGCCGACCTGTCGCGCTCGGCCGTGGTGCTGCCCGACCACCCCAATGGCATGGTCGT 4320
     eMetAlaSerAlaGlyTyrProPheThrThrTyrAlaAspLeuSerArgSerAlaValValLeuProAspHisProAsnGlyMetValVa

4321 CAGCGCCTATCTTGATCTCATGGGCTTCATGGGCGCGACGACATGGTATCCGGTGTCCGGCGTGGATGTGGTCTCGAGCGACCATGTAAA 4410
     lSerAlaTyrLeuAspLeuMetGlyPheMetGlyAlaThrThrTrpTyrProValSerGlyValAspValValSerSerAspHisValAs

4411 TGATGTGGCGGACCGGAACCTGATTGTCCTGTCCACGCTGGCCAATAGCGGCGATGTTTCGCAACTGCTGAGCAAATCGTCCTATCAGAT 4500
     nAspValAlaAspArgAsnLeuIleValLeuSerThrLeuAlaAsnSerGlyAspValSerGlnLeuLeuSerLysSerSerTyrGlnIl
                                                                                  MK172
4501 TTCTGACGGGCGGCTGCACATGGGGCTGCGCTCGACGCTGAGCGGCGTATGGAACCTGTTCCAGGATCCCATGTCGGGCATCAGCAATAC 4590
     eSerAspGlyArgLeuHisMetGlyLeuArgSerThrLeuSerGlyValTrpAsnLeuPheGlnAspProMetSerGlyIleSerAsnTh

4591 GGCCCCGACCGATGTCGAGAGCACGCTGACCGGGGGGGTAGCCGCGATGATCGAGGCAGAATCGCCTCTGGCATCAGGCCGGACCGTGCT 4680
     rAlaProThrAspValGluSerThrLeuThrGlyGlyValAlaAlaMetIleGluAlaGluSerProLeuAlaSerGlyArgThrValLe

4681 CGCGCTGCTTTCGGGTGACGGGCAGGGGCTCAACAATCTTGTGCAGATCCTCGCACAGCGTAAAAACCAGGCCAAGATCCAGGGCGACCT 4770
     uAlaLeuLeuSerGlyAspGlyGlnGlyLeuAsnAsnLeuValGlnIleLeuAlaGlnArgLysAsnGlnAlaLysIleGlnGlyAspLe

4771 TGTGCTGGCGCATGGCGATGATCTGACATCCTACCGGAGTTCGCCGCTGTATACGGTTGGCACCGTGCCGCTGTGGCTCGAGCCTGACTG 4860
     uValLeuAlaHisGlyAspAspLeuThrSerTyrArgSerSerProLeuTyrThrValGlyThrValProLeuTrpLeuGluProAspTr

4861 GTATATGCACAACCACCCCAGCCGCGTGATCGTGGTGGGCCTGCTCGGGTGCATTCTGATTGTGGCCGTCATGGTGCGCGCCCTGGCCAA 4950
     pTyrMetHisAsnHisProSerArgValIleValValGlyLeuLeuGlyCysIleLeuIleValAlaValMetValArgAlaLeuAlaLy
                                                      Gene B ——>|      |——>Gene C
4951 GCATGCTCTGCGCCGCCGTCGTGAGCTGCAGGAAGAAAGGCAGAGAACGTGATCATGAACAGGCGATACGTCCTTTCGCTTTCTGGTGCC 5040
     sHisAlaLeuArgArgArgArgGluLeuGlnGluGluArgGlnArgThr     MetAsnArgArgTyrValLeuSerLeuSerGlyAla

5041 CTGCTGGCCAGCAGTTGCATGACGGTGCTGGTGGCGGTTCCTGTTGCGCGGGCGCAGCAGGCTTCCACCGCCATGACCACCGCTGCCACG 5130
     LeuLeuAlaSerSerCysMetThrValLeuValAlaValProValAlaArgAlaGlnGlnAlaSerThrAlaMetThrThrAlaAlaThr

5131 AGCGCGACTGCGGCACCACGGCAGATCCTGTTGCAGCAGGCACGCTTCTGGCTTCAGCAGCAGCAGTATGACAATGCCCGCCAGGCCTTG 5220
     SerAlaThrAlaAlaProArgGlnIleLeuLeuGlnGlnAlaArgPheTrpLeuGlnGlnGlnGlnTyrAspAsnAlaArgGlnAlaLeu
```

FIG. 1-6

5221 CAGAACGCGGAGCGCATCGCCCCCAATTCCCCTGACGTGCTGGAAGTGCTGGGTGAATACCAGACGGCCATTGGCAACCGCGAAGCCGCC 5310
GlnAsnAlaGluArgIleAlaProAsnSerProAspValLeuGluValLeuGlyGluTyrGlnThrAlaIleGlyAsnArgGluAlaAla

5311 GCCGATACGCTGCGCCACCTGCAGCAGGTGGCGCCGGGCAGTGCCGCGGCAGGTAACCTGAATGACCTGCTCAGCGAGCGGGCCATCTCC 5400
AlaAspThrLeuArgHisLeuGlnGlnValAlaProGlySerAlaAlaAlaGlyAsnLeuAsnAspLeuLeuSerGluArgAlaIleSer

5401 CAAAGCGACCTGTCGCAGATCCGCTCGCTGGCGGGTTCGGGCCAGAACGCGCAGGCGGTGGCGGGCTACCAGAAGCTGTTCCACGGTGGC 5490
GlnSerAspLeuSerGlnIleArgSerLeuAlaGlySerGlyGlnAsnAlaGlnAlaValAlaGlyTyrGlnLysLeuPheHisGlyGly

5491 AAGCCGCCGCATTCGCTCGCGGTGGAATACTACCAGACCATGGCGGGCGTGCCGGCCCAGTGGGACCAGGCCCGCGCCGGGCTTGCCGGG 5580
LysProProHisSerLeuAlaValGluTyrTyrGlnThrMetAlaGlyValProAlaGlnTrpAspGlnAlaArgAlaGlyLeuAlaGly

5581 GTCGTTGCGTCAAACCCGCAGGATTACCGCGCCCAGCTCGCCTTTGCCCAGGCCCTGACCTATAATACCTCGACCCGCATGGAAGGCCTG 5670
ValValAlaSerAsnProGlnAspTyrArgAlaGlnLeuAlaPheAlaGlnAlaLeuThrTyrAsnThrSerThrArgMetGluGlyLeu

5671 ACCCGGCTCAAGGATCTCCAGTCCTTCCGCAGCCAGGCCCCGGTCGAGGCGGCCGCCGCGGCGCAGTCCTACCGCCAGACCCTGAGCTGG 5760
ThrArgLeuLysAspLeuGlnSerPheArgSerGlnAlaProValGluAlaAlaAlaAlaAlaGlnSerTyrArgGlnThrLeuSerTrp

5761 CTGCCGGTCAATCCTGAGACGCAGCCCCTCATGGAGCAGTGGCTTTCCGCCCACCCCAATGATACCGCGCTGCGCGAGCATATGCTCCAC 5850
LeuProValAsnProGluThrGlnProLeuMetGluGlnTrpLeuSerAlaHisProAsnAspThrAlaLeuArgGluHisMetLeuHis

5851 CCCCCCGGTGGTCCGCCGGACAAGGCCGGGCTTGCGCGCCAGGCAGGTTACCAGCAGCTTAACGCGGGCCGTCTTGCCGCAGCCGAGCAG 5940
ProProGlyGlyProProAspLysAlaGlyLeuAlaArgGlnAlaGlyTyrGlnGlnLeuAsnAlaGlyArgLeuAlaAlaAlaGluGln

5941 TCTTTCCAGTCGGCGTTGCAGATCAATTCCCATGATGCTGATTCGCTTGGTGGCATGGGGCTCGTAAGCATGCGGCAGGGCGATACCGCG 6030
SerPheGlnSerAlaLeuGlnIleAsnSerHisAspAlaAspSerLeuGlyGlyMetGlyLeuValSerMetArgGlnGlyAspThrAla

6031 GAGGCGCGCCGCTATTTTGAAGAAGCGATGGCCGCCGACCCCAAGACCGCCGATCGCTGGCGCCCGGCGCTTGCGGGCATGGCCGTCAGC 6120
GluAlaArgArgTyrPheGluGluAlaMetAlaAlaAspProLysThrAlaAspArgTrpArgProAlaLeuAlaGlyMetAlaValSer

6121 GGCGAGTATGCTTCCGTTCGCCAGTTGATTGCCGCCCATCAATATACCGAGGCCAAGCAGCAGCTTGCCACGCTGGCCCGCCAGCCCGGC 6210
GlyGluTyrAlaSerValArgGlnLeuIleAlaAlaHisGlnTyrThrGluAlaLysGlnGlnLeuAlaThrLeuAlaArgGlnProGly

FIG. 1-7

```
6211 CAGTATACTGGCGCGACCCTCATGCTGGCCGACCTGCAGCGCTCGACCGGCCAGATTGCCGCCGCCGAGCAGGAATATCGTGGCATCCTG 6300
     GlnTyrThrGlyAlaThrLeuMetLeuAlaAspLeuGlnArgSerThrGlyGlnIleAlaAlaAlaGluGlnGluTyrArgGlyIleLeu

6301 TCGCGTGAGCCCAATAACCAGTTGGCCCTCATGGGGCTGGCCCGGGTAGACATGGCGCAGGGCAACACGGCGGAAGCACGCCAGCTCCTG 6390
     SerArgGluProAsnAsnGlnLeuAlaLeuMetGlyLeuAlaArgValAspMetAlaGlnGlyAsnThrAlaGluAlaArgGlnLeuLeu

6391 TCGCGTGTCGGCCCGCAATATGCAAGCCAGGTGGGCGAGATCGAGGTTTCGGGCCTGATGGCGGCTGCGTCCCAGACATCGGATTCAGCG 6480
     SerArgValGlyProGlnTyrAlaSerGlnValGlyGluIleGluValSerGlyLeuMetAlaAlaAlaSerGlnThrSerAspSerAla

6481 CGCAAGGTTTCCATCCTGCGCGAAGCGATGGCCCAGGCCCCACGTGACCCCTGGGTGCGCATCAACCTTGCCAATGCGCTGCAGCAGCAG 6570
     ArgLysValSerIleLeuArgGluAlaMetAlaGlnAlaProArgAspProTrpValArgIleAsnLeuAlaAsnAlaLeuGlnGlnGln

6571 GGCGACGTGGCCGAAGCCGGGCGCGTGATGCAGCCCATCCTGGCCAATCCCGTCACCGCGCAGGACCGCCAGGCCGGTATCCTTTATACC 6660
     GlyAspValAlaGluAlaGlyArgValMetGlnProIleLeuAlaAsnProValThrAlaGlnAspArgGlnAlaGlyIleLeuTyrThr

6661 TATGGTAGTGGCAATGATGCGATGACCCGCCAGCTTCTGGCTGGTCTGTCGCCTGCGGATTATTCTCCTGCCATCCGTTCCATCGCCGAG 6750
     TyrGlySerGlyAsnAspAlaMetThrArgGlnLeuLeuAlaGlyLeuSerProAlaAspTyrSerProAlaIleArgSerIleAlaGlu

6751 GAAATGGAAATCAAGCAGGATCTGGCCAGCCGCCTGTCCATGGTGTCCAACCCGGTGCCGCTGATCCGCGAGGCCCTGACCCAGCCTGAT 6840
     GluMetGluIleLysGlnAspLeuAlaSerArgLeuSerMetValSerAsnProValProLeuIleArgGluAlaLeuThrGlnProAsp

6841 CCGACCGGCGCGCGCGGCGTGGCGGTGGCTGACCTGTTCCGCCAGCGTGGCGACATGGTGCATGCCCGCATGGCACTGCGTATCGCCTCG 6930
     ProThrGlyAlaArgGlyValAlaValAlaAspLeuPheArgGlnArgGlyAspMetValHisAlaArgMetAlaLeuArgIleAlaSer

6931 ACGCGCACCATCGATCTCTCGCCCGACCAGCGCCTGTCCTATGCCACCGAATACATGAAGATCAGCAACCCGGTGGCCGCTGCGCGGCTG 7020
     ThrArgThrIleAspLeuSerProAspGlnArgLeuSerTyrAlaThrGluTyrMetLysIleSerAsnProValAlaAlaAlaArgLeu

7021 CTGGCCCCGCTGGGGGATGGCACGGGCTCGGCTACAGGAAGCGCGTTGCTGCCCGAGCAGGTGCAGACGCTCCAGCAACTGCGCATGGGC 7110
     LeuAlaProLeuGlyAspGlyThrGlySerAlaThrGlySerAlaLeuLeuProGluGlnValGlnThrLeuGlnGlnLeuArgMetGly

7111 ATCTCGGTGGCGCAGTCCGATCTGCTCAACCAGCGTGGCGACCAGGCGCAGGCCTATGATCATCTGGCCCCCGCGCTGCAGGCCGACCCG 7200
     IleSerValAlaGlnSerAspLeuLeuAsnGlnArgGlyAspGlnAlaGlnAlaTyrAspHisLeuAlaProAlaLeuGlnAlaAspPro

7201 GAGGCGACATCGCCCAAGCTGGCGCTCGCGCGGCTGTATAATGGCCACGGCAAGCCGGGCAAGGCGCTCGAGATCGACCTTGCGGTGCTG 7290
     GluAlaThrSerProLysLeuAlaLeuAlaArgLeuTyrAsnGlyHisGlyLysProGlyLysAlaLeuGluIleAspLeuAlaValLeu
```

FIG. I-8

```
7291 CGCCACAACCCGCAGGACCTTGATGCGCGACAGGCTGCGGTGCAGGCGGCGGTCAACAGCGACCACAACAGCCTTGCCACCCGCCTTGCC 7380
     ArgHisAsnProGlnAspLeuAspAlaArgGlnAlaAlaValGlnAlaAlaValAsnSerAspHisAsnSerLeuAlaThrArgLeuAla

7381 ATGGATGGCGTGCAGGAAAGCCCGATGGATGCCCGTGCCTGGCTGGCCATGGCCGTGGCTGACCAGGCCGATGGCCACGGGCAGCGCACC 7470
     MetAspGlyValGlnGluSerProMetAspAlaArgAlaTrpLeuAlaMetAlaValAlaAspGlnAlaAspGlyHisGlyGlnArgThr

7471 ATCGAGGATCTGCGCCGCGCCTATGACCTGCGCCTGCAGCAGGTCGAGGGCACGCGGGCCGCGTCTGGCGCGGGTGCTGCGCAGGAAGAT 7560
     IleGluAspLeuArgArgAlaTyrAspLeuArgLeuGlnGlnValGluGlyThrArgAlaAlaSerGlyAlaGlyAlaAlaGlnGluAsp

7561 GCGCTTGCTCCGCCCTCGACCAACCCGTTCCGCCCGCGTGGCTACGGCCACCAGACGGAACTTGGCGCGCCTGTGACCGGTGGCTCCTAC 7650
     AlaLeuAlaProProSerThrAsnProPheArgProArgGlyTyrGlyHisGlnThrGluLeuGlyAlaProValThrGlyGlySerTyr

7651 AGCGCCGAGGCGGCATCGCCCGATACGTCGGACCAGATGCTCTCCTCCATCGCAGGCCAGATCCGCACGCTGCGTGAGAACCTTGCCCCT 7740
     SerAlaGluAlaAlaSerProAspThrSerAspGlnMetLeuSerSerIleAlaGlyGlnIleArgThrLeuArgGluAsnLeuAlaPro

7741 TCCATCGATGGTGGCCTCGGGTTCCGCTCGCGTTCGGGTGAGCATGGCATGGGCCGCCTGACGGAAGCGAACATTCCCATCGTGGGCCGC 7830
     SerIleAspGlyGlyLeuGlyPheArgSerArgSerGlyGluHisGlyMetGlyArgLeuThrGluAlaAsnIleProIleValGlyArg

7831 CTGCCGCTGCAGGCCGGTGCTTCCGCCCTGACCTTCTCGATCACGCCAACCATGATCTGGTCGGGCAACCTCAACACGGGTTCCGTCTAT 7920
     LeuProLeuGlnAlaGlyAlaSerAlaLeuThrPheSerIleThrProThrMetIleTrpSerGlyAsnLeuAsnThrGlySerValTyr

7921 GATGTGCCGCGTTATGGCACGATGATGGGCGTGCAGGCATATAACCAGTACGATAGCTATACCAACGCGGGCAGGGACCAGCAGCGCATC 8010
     AspValProArgTyrGlyThrMetMetGlyValGlnAlaTyrAsnGlnTyrAspSerTyrThrAsnAlaGlyArgAspGlnGlnArgIle

8011 GCCGCTGGCACGGCCGAGGCCGGGTTTGCGCCGGATGTGCAGTTTGGCAATAGCTGGGTGCGGGCCGATGTGGGTGCGTCGCCCATCGGC 8100
     AlaAlaGlyThrAlaGluAlaGlyPheAlaProAspValGlnPheGlyAsnSerTrpValArgAlaAspValGlyAlaSerProIleGly

8101 TTCCCCATCACCAACGTGCTGGGCGGTGTCGAGTTCTCGCCGCGCGTGGGTCCGGTCACCTTCCGTGTCAGTGCCGAGCGCCGGTCGATC 8190
     PheProIleThrAsnValLeuGlyGlyValGluPheSerProArgValGlyProValThrPheArgValSerAlaGluArgArgSerIle

8191 ACCAACAGCGTGCTGTCCTATGGCGGCCTGCGTGACACGAACTACAACAGCGCGCTTGGCCGGTATGCCCGCCAGGTCTACGGCCAGGCA 8280
     ThrAsnSerValLeuSerTyrGlyGlyLeuArgAspThrAsnTyrAsnSerAlaLeuGlyArgTyrAlaArgGlnValTyrGlyGlnAla
```

FIG. 1-9

```
8281 CTGTCCAAGCAGTGGGGCAGCGAATGGGGTGGCGTCGTGACCAACCACTTCCATGGGCAGGTCGAGGCGACACTGGGCAACACCATCCTG 8370
     LeuSerLysGlnTrpGlySerGluTrpGlyGlyValValThrAsnHisPheHisGlyGlnValGluAlaThrLeuGlyAsnThrIleLeu

8371 TATGGTGGCGGTGGCTACGCAATCCAGACCGGCAAGAACGTGCAGCGCAACAGCGAGCGTGAAGCGGGCATCGGCGCCAATACGCTGGTG 8460
     TyrGlyGlyGlyGlyTyrAlaIleGlnThrGlyLysAsnValGlnArgAsnSerGluArgGluAlaGlyIleGlyAlaAsnThrLeuVal

8461 TGGCATAACGCCAACATGCTGGTGCGCATTGGCGTGAGCCTGACCTATTTCGGTTATGCCAAGAACGAGGATTTCTACACCTACGGCCAG 8550
     TrpHisAsnAlaAsnMetLeuValArgIleGlyValSerLeuThrTyrPheGlyTyrAlaLysAsnGluAspPheTyrThrTyrGlyGln

8551 GGTGGTTACTTCTCGCCGCAATCCTATTACGCGGCGACCGTGCCGGTGCGCTATGCGGGCCAGCACAAGCGGCTGGACTGGGACGTGACG 8640
     GlyGlyTyrPheSerProGlnSerTyrTyrAlaAlaThrValProValArgTyrAlaGlyGlnHisLysArgLeuAspTrpAspValThr

8641 GGCAGCGTGGGCTACCAGGTGTTCCACGAGCACTCGGCGCCCTTCTTCCCCACGTCATCGCTGCTGCAGTCCGGCGCCAATACCATCGCG 8730
     GlySerValGlyTyrGlnValPheHisGluHisSerAlaProPhePheProThrSerSerLeuLeuGlnSerGlyAlaAsnThrIleAla

873  TCGAATTACTCGGCGAGCGCCACGCCGGCGGAATATCTGTCGGAGGAAACGGTGAACAGCGCCTACTATCCTGGGGATAGTATTGCTGGT 8820
     SerAsnTyrSerAlaSerAlaThrProAlaGluTyrLeuSerGluGluThrValAsnSerAlaTyrTyrProGlyAspSerIleAlaGly

8821 CTTACCGGTGGCTTTAATGCTAGGGTGGGTTATCGCTTTACACGCAATGTTCGTCTTGATCTCTCGGGGCGCTATCAGAAGGCCGGTAAC 8910
     LeuThrGlyGlyPheAsnAlaArgValGlyTyrArgPheThrArgAsnValArgLeuAspLeuSerGlyArgTyrGlnLysAlaGlyAsn
                                                    Gene C ——>|  |——> Gene D
8911 TGGACTGAAAGCGGCGCCATGATTTCCGCACACTATCTTATTATGGACCAGTAATGACAACTTTGAACGCAAAACCGGACTTTTCGCTTT 9000
     TrpThrGluSerGlyAlaMetIleSerAlaHisTyrLeuIleMetAspGln  MetThrThrLeuAsnAlaLysProAspPheSerLeuP

9001 TCCTGCAGGCCCTGTCCTGGGAGATCGATGATCAGGCCGGGATCGAGGTCCGCAATGACCTGTTGCGCGAGGTCGGCCGTGGTATGGCTG 9090
     heLeuGlnAlaLeuSerTrpGluIleAspAspGlnAlaGlyIleGluValArgAsnAspLeuLeuArgGluValGlyArgGlyMetAlaG

9091 GTCGTTTCCAGCCGCCGCTGTGCAACACCATCCACCAGCTCCAGATCGAGCTGAACGCCCTGCTGGCCATGATCAACTGGGGCTACGTGA 9180
     lyArgPheGlnProProLeuCysAsnThrIleHisGlnLeuGlnIleGluLeuAsnAlaLeuLeuAlaMetIleAsnTrpGlyTyrValL
```

FIG. 1-10

```
9181 AGCTGGACCTGCTGGCGGAAGAACAGGCCATGCGCATCGTGCATGAAGACCTGCCTCAGGTGGGCAGCGCAGGCGAGCCCGCCGGCACGT 9270
     ysLeuAspLeuLeuAlaGluGluGlnAlaMetArgIleValHisGluAspLeuProGlnValGlySerAlaGlyGluProAlaGlyThrT

9271 GGCTTGCCCCGGTTCTGGAAGGGCTTTATGGCCGCTGGATCACGTCGCAGCCCGGTGCATTTGGTGATTACGTCGTGACGCGCGATATCG 9360
     rpLeuAlaProValLeuGluGlyLeuTyrGlyArgTrpIleThrSerGlnProGlyAlaPheGlyAspTyrValValThrArgAspIleA
                                                                                Gene D ---->|
9361 ACGCGGAAGACCTGAACTCGGTTCCGGCCCAGACGATCATCCTTTACATGCGCACCCGCAGCGCCGCGACCTGATTCCTGCCAGTCGCGC 9450
     spAlaGluAspLeuAsnSerValProAlaGlnThrIleIleLeuTyrMetArgThrArgSerAlaAlaThr

9451 CATTTGCGTCAAAACCCTGCCTACAGGCGTGTTCATGCCCTGTAGGCAGGGTTTTTGCATATAGGGTTCCACTCTTTGCCCTGTTTTTGC 9540

9541 GCTAGATCATGCGGCGTGGGAGCAGGGTGCTTCACAAATGGGTCAAGAAATGGCGGACGCAGGCCGCCGCGCCGTCACGATCCAGCCCCT 9630

9631 GAAGGAAGAGCCAGCCACATGAAACTGTCCCGCAAGATATTCCTGTTATCCGCCGTGGCGTGTGGCATGGCCCTGGCCCAGGCACCCGCT 9720
                           MetLysLeuSerArgLysIlePheLeuLeuSerAlaValAlaCysGlyMetAlaLeuAlaGlnAlaProAla

9721 TTTGCGCGGCATGCGCATGATGGTGGAGGCGACCCGGCCGATAGTCGGGCGCGGCAACTGCTCGCCACCATGAGCCTTGAGGACAAGATG 9810
     PheAlaArgHisAlaHisAspGlyGlyGlyAspProAlaAspSerArgAlaArgGlnLeuLeuAlaThrMetSerLeuGluAspLysMet

9811 TCCCTGCTGTTCAGCGTTGATGGTGGTGGCTTCAATGGCAGCGTGGCGCCTCCGGGGGGGCTGGGTTCGGCGGCTTACCTGCGCGCGCCC 9900
     SerLeuLeuPheSerValAspGlyGlyGlyPheAsnGlySerValAlaProProGlyGlyLeuGlySerAlaAlaTyrLeuArgAlaPro

9901 AAGGGGTCGGGCCTGCCAGACCTGCAGATTTCGGATGCAGGGCTTGGCGTGCGCAACCCGGCGCATATCCGCAAGAATGGCGCGGCGGTC 9990
     LysGlySerGlyLeuProAspLeuGlnIleSerAspAlaGlyLeuGlyValArgAsnProAlaHisIleArgLysAsnGlyAlaAlaVal

9991 TCCCTGCCGTCCGGGCTGTCCACGGCCAGCACGTGGGATATGGACATGGCGCGGCAGGCCGGTGTCATGATCGGGCGCGAAGCGTGGCAG 10080
     SerLeuProSerGlyLeuSerThrAlaSerThrTrpAspMetAspMetAlaArgGlnAlaGlyValMetIleGlyArgGluAlaTrpGlr

10081 AGCGGCTTCAACATCCTGCTTGGCGGCGGCGCCGACCTGACGCGCGACCCGCGTGGCGGCCGCAACTTTGAATATGCGGGCGAGGATCC 10169
     SerGlyPheAsnIleLeuLeuGlyGlyGlyAlaAspLeuThrArgAspProArgGlyGlyArgAsnPheGluTyrAlaGlyGluAsp
```

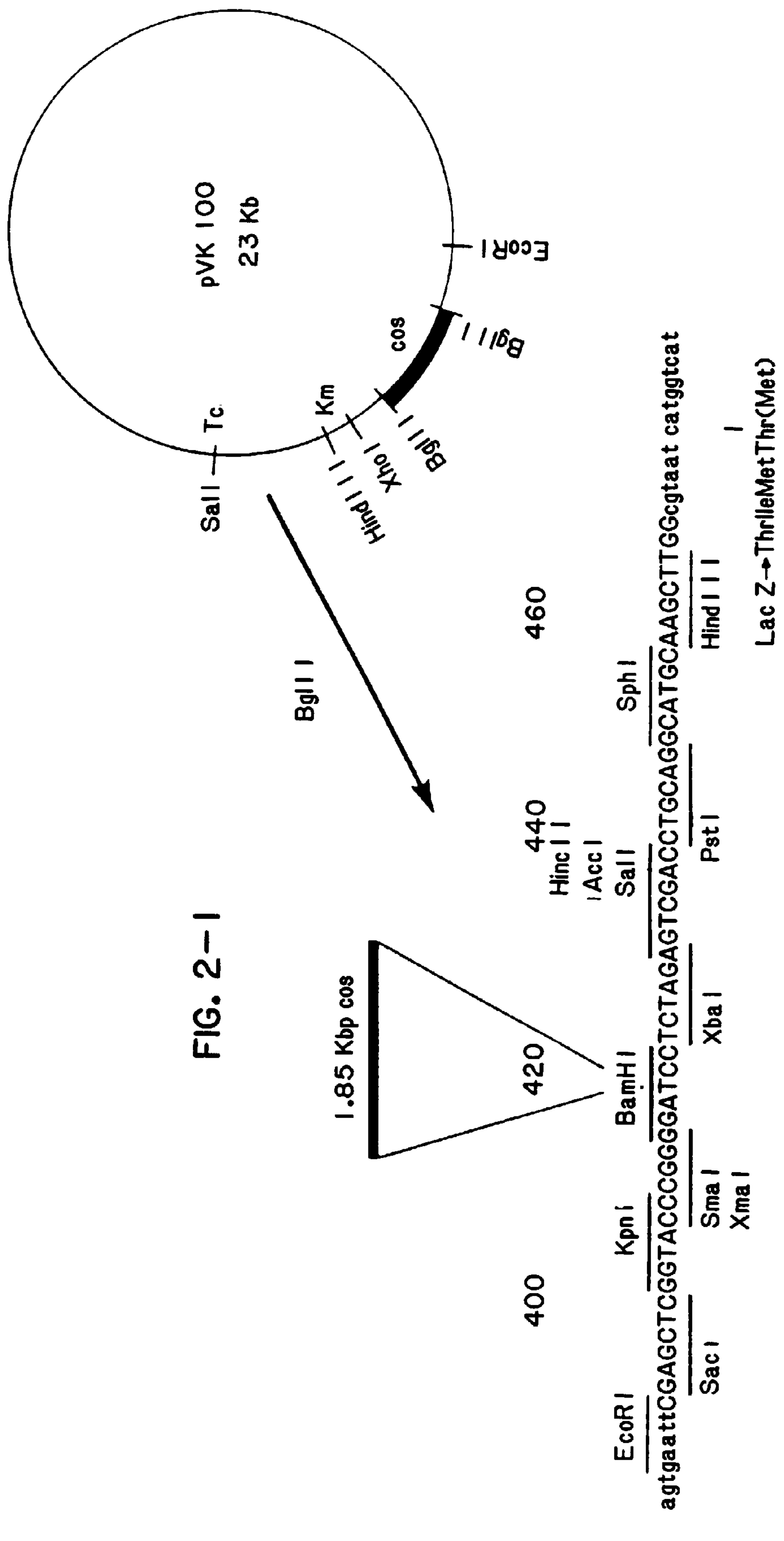
pVK 100
23 Kb
EcoRI
BglII
cos
BglII
Km
XhoI
HindIII
Tc
SalI
BglII
1.85 Kbp cos
400
420
440
460
EcoRI
KpnI
BamHI
HincII
AccI
SalI
SphI
agtgaattCGAGCTCGGTACCCGGGGATCCTCTAGAGTCGACCTGCAGGCATGCAAGCTTGGcgtaat catggtcat
SacI
SmaI
XmaI
XbaI
PstI
HindIII
Lac Z→ThrIleMetThr(Met)

FIG. 2-1

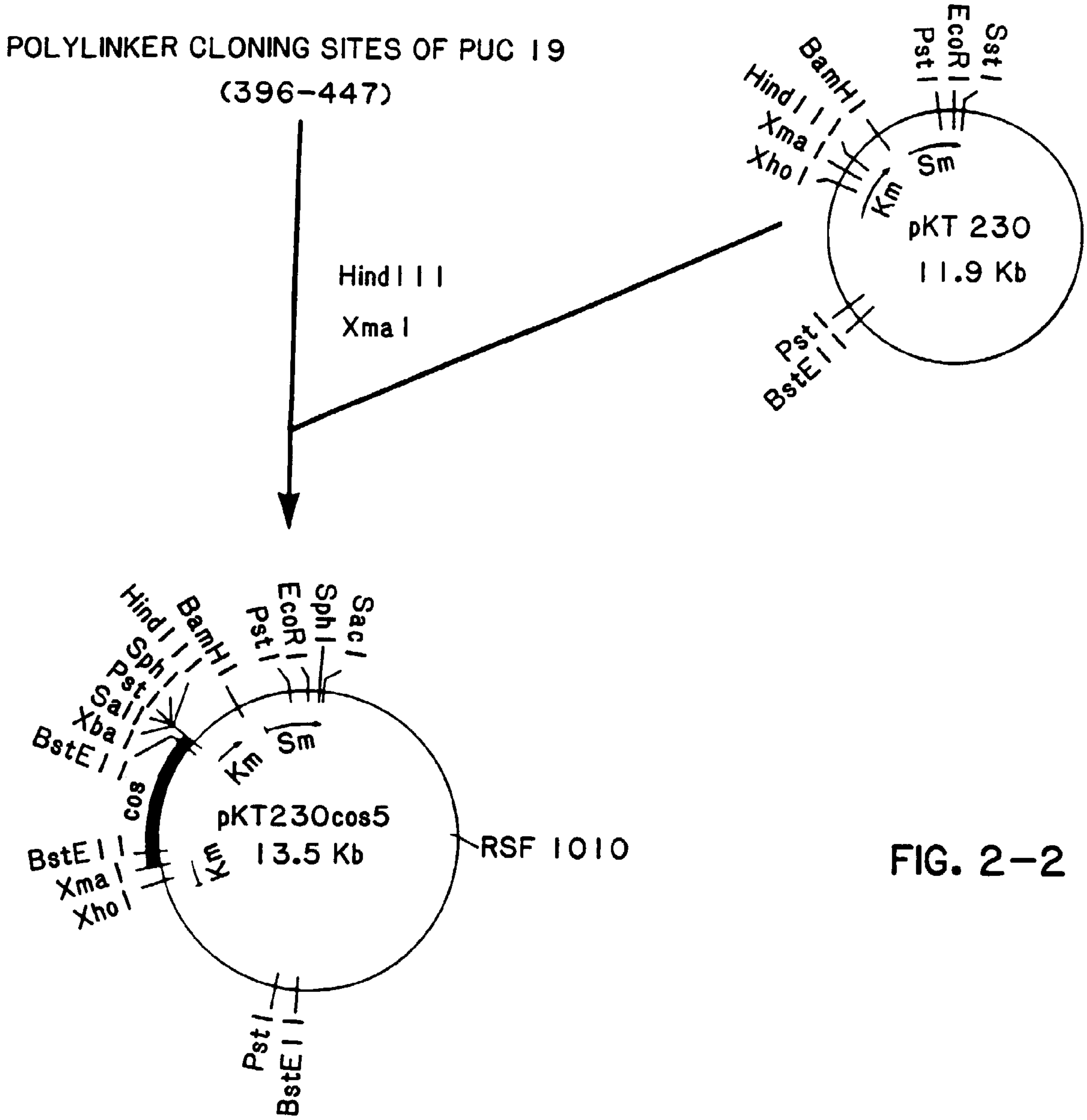
POLYLINKER CLONING SITES OF PUC 19
(396-447)
HindIII
XmaI
pKT 230
11.9 Kb
BamHI
HindIII
XmaI
XhoI
PstI
EcoRI
SstI
Sm
Km
PstI
BstEII
pKT230cos5
13.5 Kb
HindIII
SphI
PstI
SalI
XbaI
BstEII
BamHI
EcoRI
SphI
SacI
cos
Km
Sm
RSF 1010
XmaI
XhoI

FIG. 2-2

FIG. 3

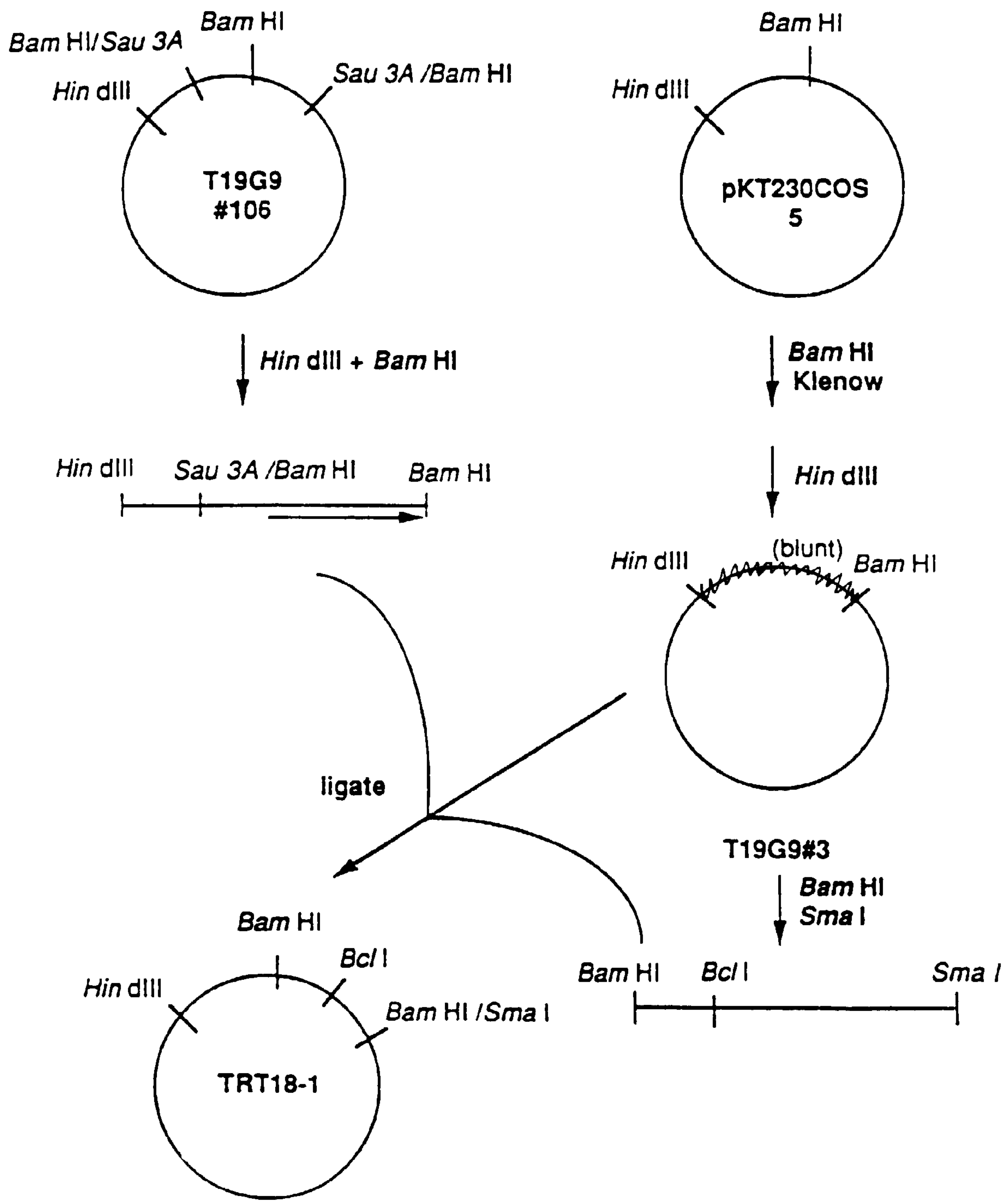
Bam HI/Sau 3A
Bam HI
Sau 3A /Bam HI
Hin dIII
T19G9
#106
Bam HI
Hin dIII
pKT230COS
5
Hin dIII + Bam HI
Bam HI
Klenow
Hin dIII
Hin dIII
Sau 3A /Bam HI
Bam HI
Hin dIII
(blunt)
Bam HI
ligate
T19G9#3
Bam HI
Sma I
Bam HI
Bcl I
Hin dIII
Bam HI /Sma I
TRT18-1
Bam HI
Bcl I
Sma I

FIG. 4

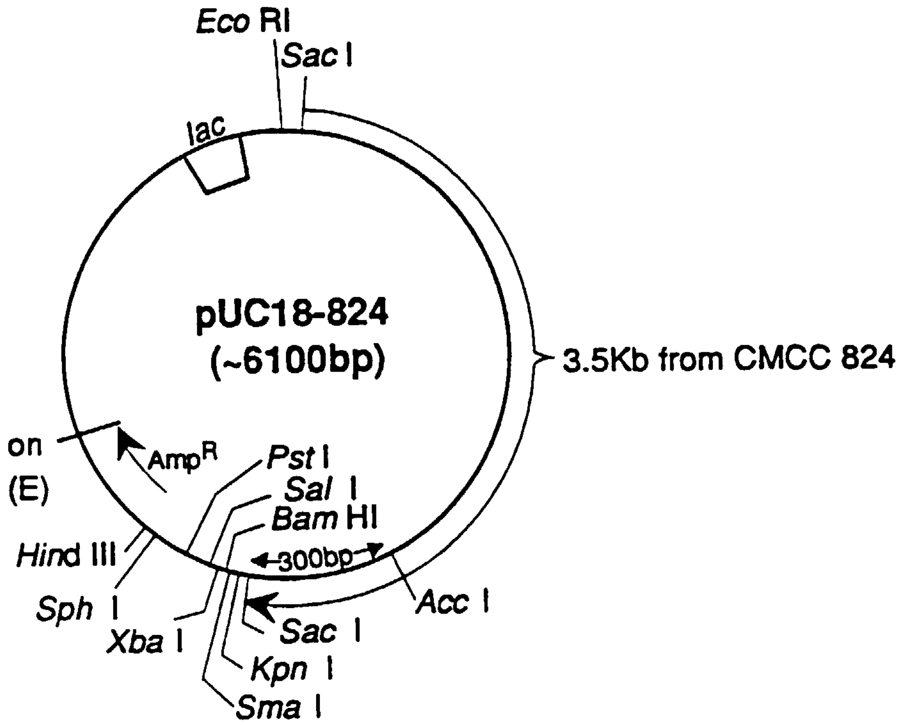
Eco RI
Sac I
lac
pUC18-824
(~6100bp)
3.5Kb from CMCC 824
on
(E)
AmpR
Pst I
Sal I
Bam HI
Hind III
300bp
Acc I
Sph I
Sac I
Xba I
Kpn I
Sma I

FIG. 7

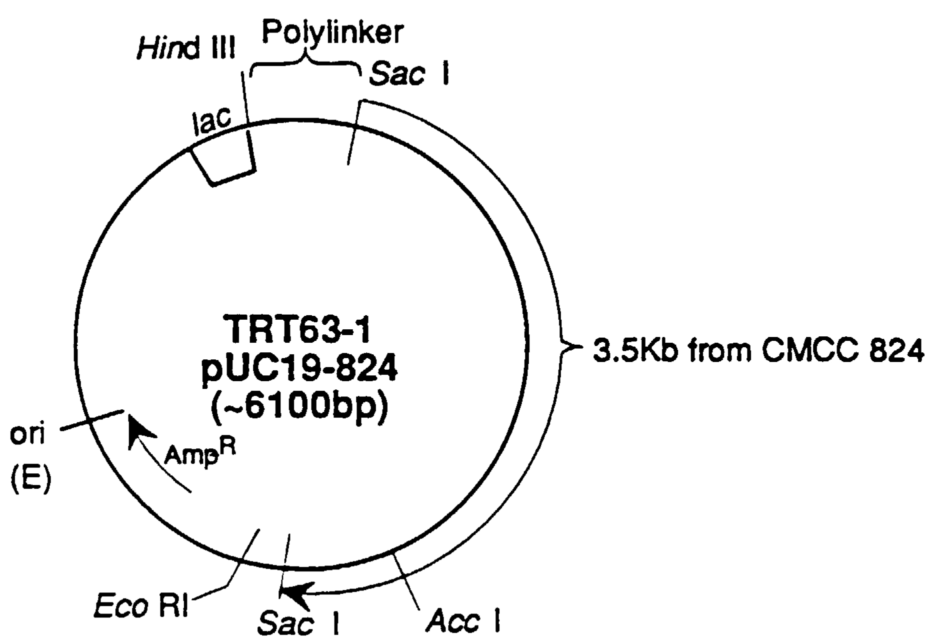
Polylinker
Hind III
Sac I
lac
TRT63-1
pUC19-824
(~6100bp)
3.5Kb from CMCC 824
ori
(E)
AmpR
Eco RI
Sac I
Acc I

FIG. 5

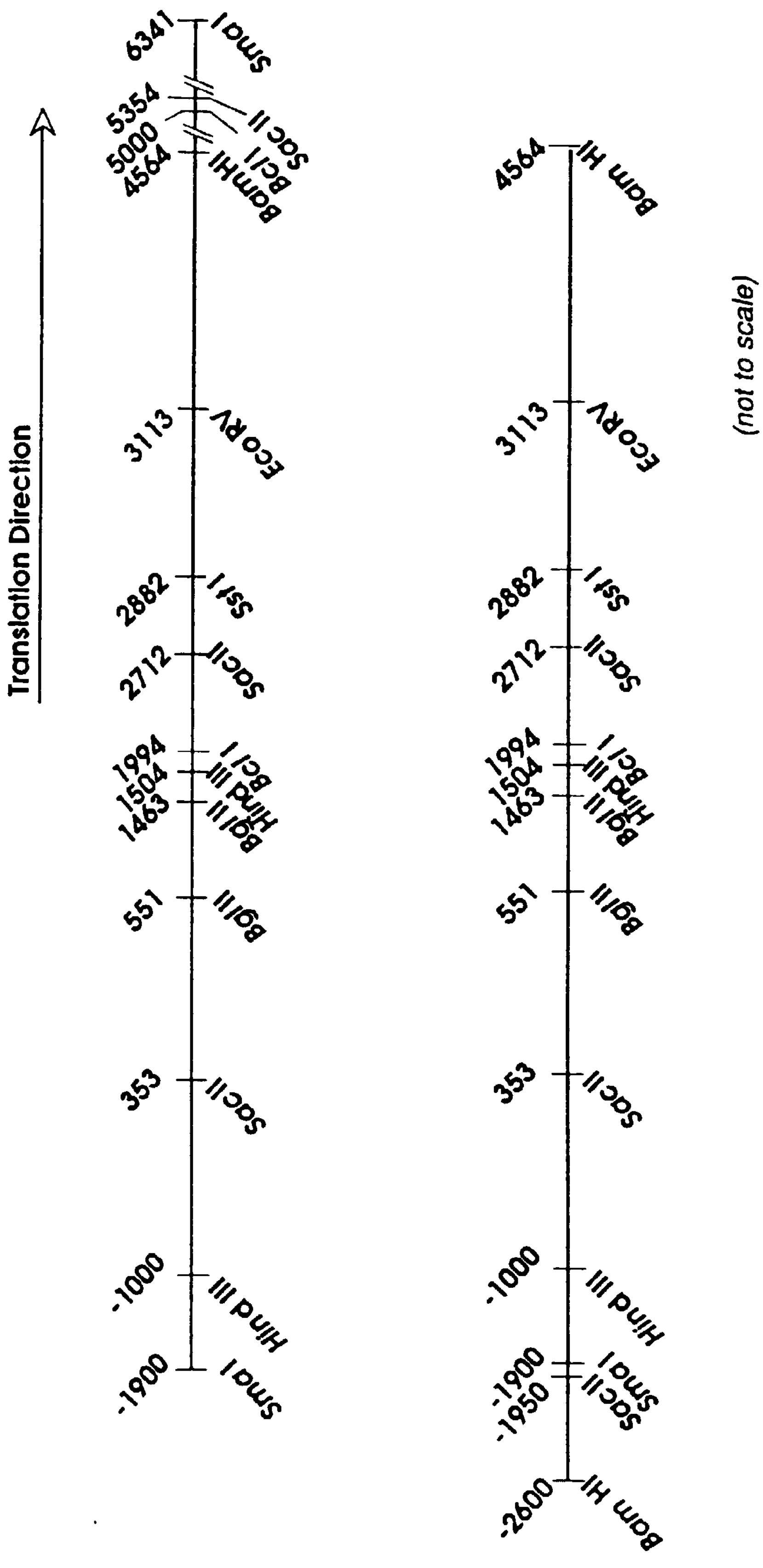
Translation Direction
6341 SmaI
5354 SacII
5000 BclI
4564 BamHI
3113 EcoRV
2882 SstI
2712 SacII
1994 BclI
1504 HindIII
1463 BglII
551 BglII
353 SacII
-1000 HindIII
-1900 SmaI
4564 Bam HI
3113 EcoRV
2882 SstI
2712 SacII
1994 BclI
1504 HindIII
1463 BglII
551 BglII
353 SacII
-1000 HindIII
-1900 SmaI
-1950 SacII
-2600 Bam HI
(not to scale)

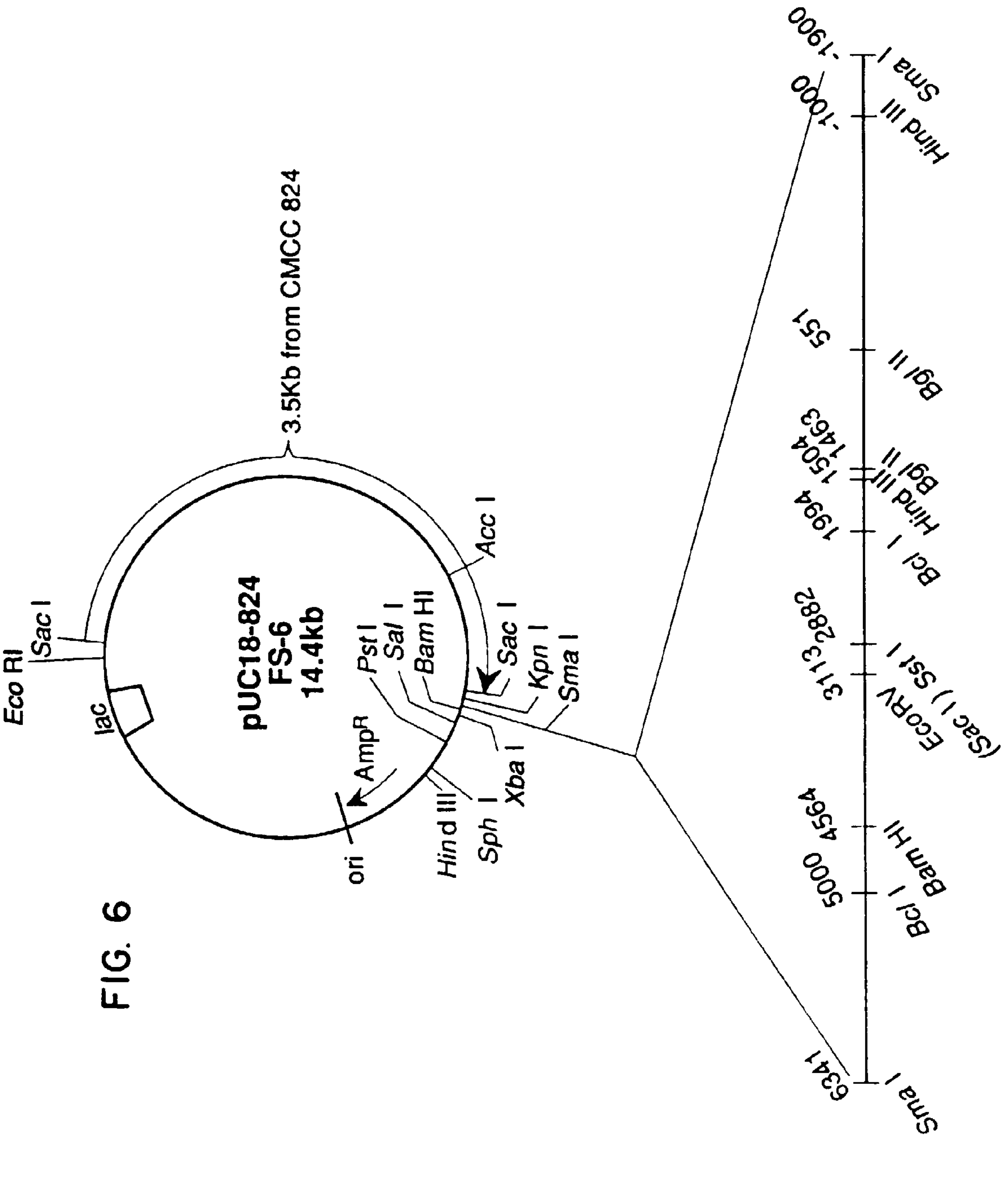
pUC18-824
FS-6
14.4kb
3.5Kb from CMCC 824
Eco RI
Sac I
lac
AmpR
ori
Hind III
Sph I
Xba I
Pst I
Sal I
Bam HI
Acc I
Sac I
Kpn I
Sma I
Sma I
Hind III
Bgl II
Hind III
Bgl II
Bcl I
Ssl I
(Sac I)
EcoRV
Bam HI
Bcl I
Sma I
-1900
-1000
551
1463
1504
1994
2882
3113
4564
5000
6341

FIG. 6

FIG. 8

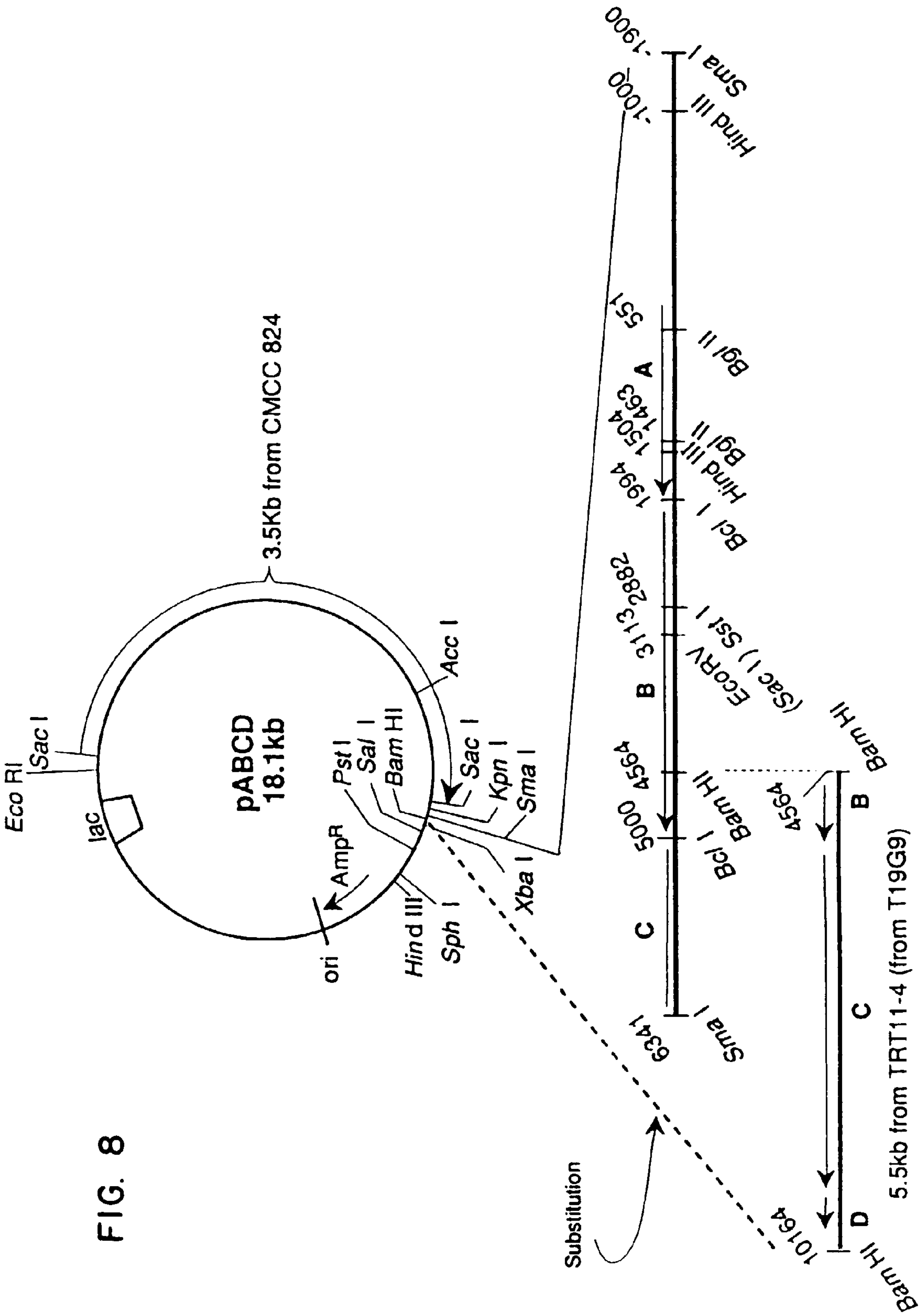
pABCD
18.1kb
3.5Kb from CMCC 824
Eco RI
Sac I
lac
AmpR
ori
Hind III
Sph I
Xba I
Pst I
Sal I
Bam HI
Sac I
Kpn I
Sma I
Acc I
Substitution
-1900
-1000
551
1463
1504
1994
2882
3113
4564
5000
6341
A
B
C
Sma I
Hind III
Bgl II
Bgl II
Hind III
Bcl I
Sst I
(Sac I)
EcoRV
Bam HI
Bcl I
Sma I
4564
10164
B
C
D
Bam HI
Bam HI
5.5kb from TRT11-4 (from T19G9)

FIG.9-1

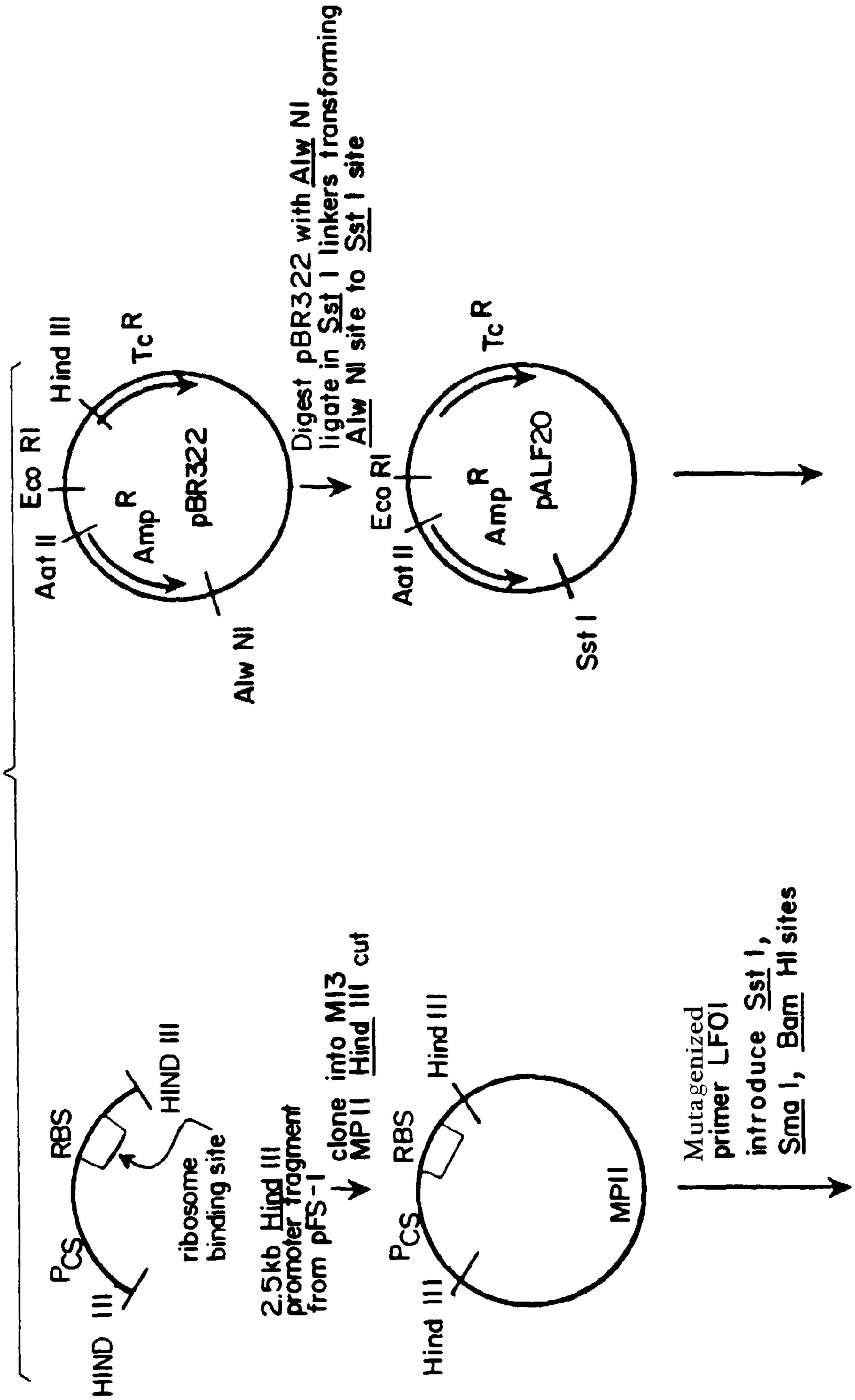
Hind III
Eco RI
Aat II
Tc R
Amp R
pBR322
Alw NI
Digest pBR322 with Alw NI
ligate in Sst I linkers transforming
Alw NI site to Sst I site
Eco RI
Aat II
Tc R
Amp R
pALF20
Sst I
HIND III
PCS
RBS
HIND III
ribosome
binding site
2.5kb Hind III
promoter fragment
from pFS-1
clone into M13
MP11 Hind III cut
Hind III
PCS
RBS
Hind III
MP11
Mutagenized
primer LF01
introduce Sst I,
Sma I, Bam HI sites

FIG.9-2

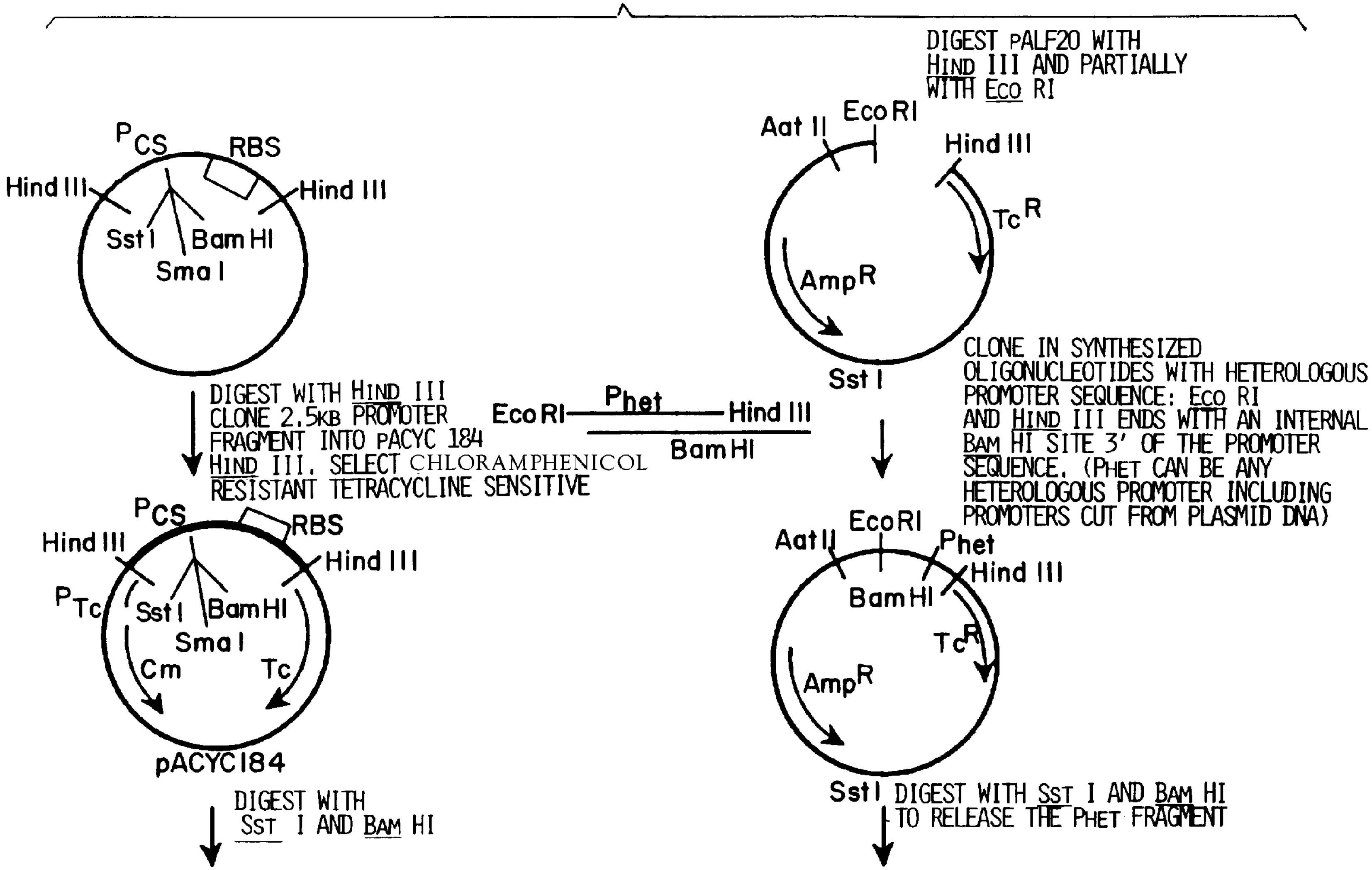
DIGEST pALF20 WITH
HIND III AND PARTIALLY
WITH ECO RI
PCS
RBS
Hind III
Hind III
Sst I
Bam HI
Sma I
Aat II
Eco RI
Hind III
TcR
AmpR
Sst I
DIGEST WITH HIND III
CLONE 2.5KB PROMOTER
FRAGMENT INTO pACYC 184
HIND III. SELECT CHLORAMPHENICOL
RESISTANT TETRACYCLINE SENSITIVE
Eco RI
Phet
Hind III
Bam HI
CLONE IN SYNTHESIZED
OLIGONUCLEOTIDES WITH HETEROLOGOUS
PROMOTER SEQUENCE: ECO RI
AND HIND III ENDS WITH AN INTERNAL
BAM HI SITE 3' OF THE PROMOTER
SEQUENCE. (PHET CAN BE ANY
HETEROLOGOUS PROMOTER INCLUDING
PROMOTERS CUT FROM PLASMID DNA)
PCS
RBS
Hind III
Hind III
PTc
Sst I
Bam HI
Sma I
Cm
Tc
pACYC184
Aat II
Eco RI
Phet
Hind III
Bam HI
TcR
AmpR
Sst I
DIGEST WITH
SST I AND BAM HI
DIGEST WITH SST I AND BAM HI
TO RELEASE THE PHET FRAGMENT

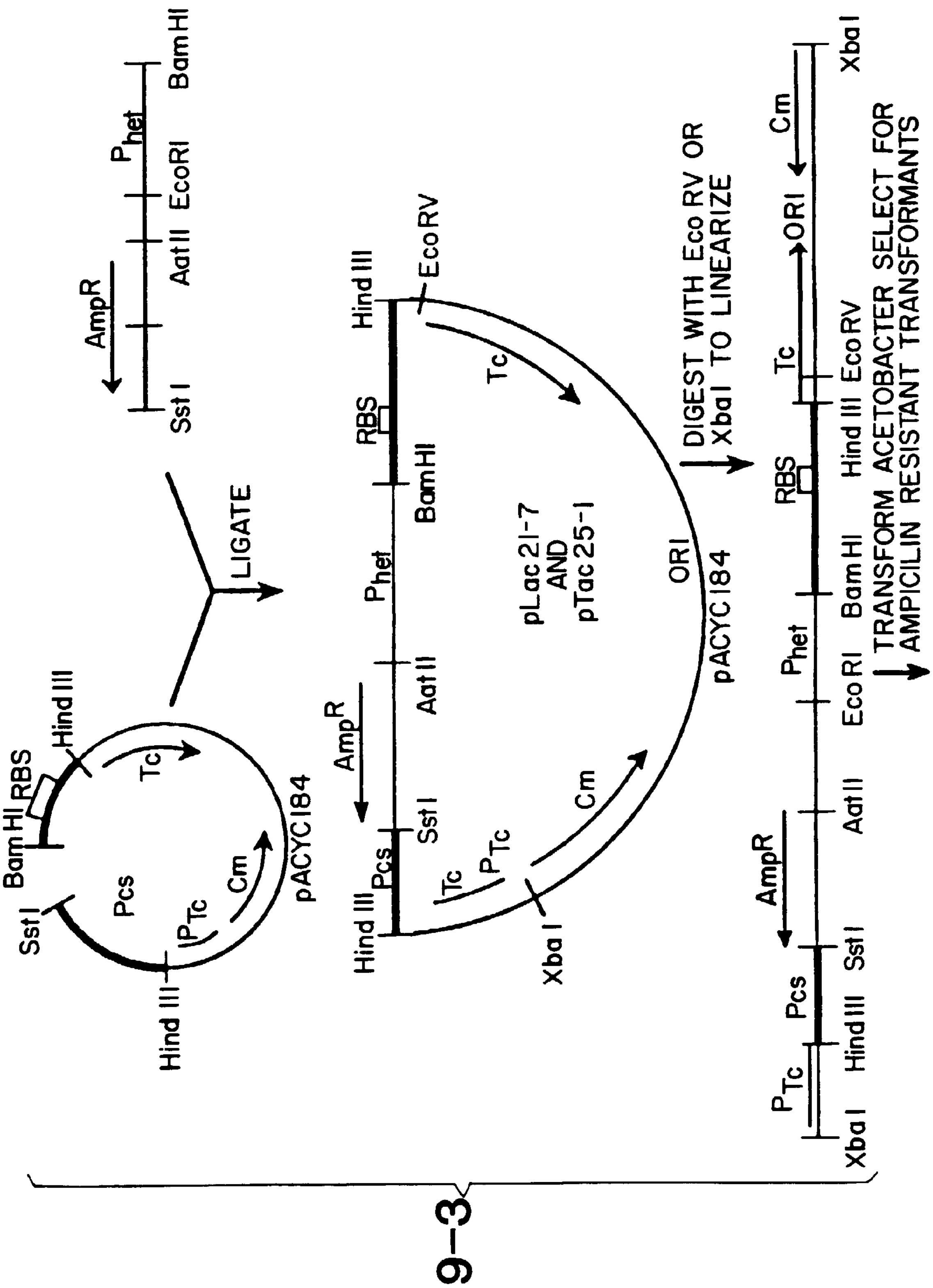
Sst I
AmpR
Aat II
EcoRI
Phet
BamHI
BamHI
RBS
Hind III
Hind III
Pcs
PTc
Tc
Cm
pACYC184
LIGATE
Hind III
Pcs
Sst I
AmpR
Aat II
Phet
BamHI
RBS
Hind III
EcoRV
Tc
pLac 21-7
AND
pTac 25-1
ORI
pACYC184
Cm
PTc
Tc
Xba I
DIGEST WITH Eco RV OR
XbaI TO LINEARIZE
Xba I
PTc
Hind III
Pcs
Sst I
AmpR
Aat II
Eco RI
Phet
Bam HI
RBS
Hind III
Tc
Eco RV
ORI
Cm
Xba I
TRANSFORM ACETOBACTER SELECT FOR
AMPICILIN RESISTANT TRANSFORMANTS

FIG.9-3